(19) Europäisches Patentamt
European Patent Office
Office européen des brevets

(11) Veröffentlichungsnummer: **0 418 199 A2**

(12) **EUROPÄISCHE PATENTANMELDUNG**

(21) Anmeldenummer: **90810668.5**

(22) Anmeldetag: **04.09.90**

(51) Int. Cl.$^5$: **C07D 213/61**, C07D 213/40, A01N 43/40, C07D 277/32, C07D 401/12

(30) Priorität: **13.09.89 CH 3335/89**
**02.04.90 CH 1078/90**

(43) Veröffentlichungstag der Anmeldung:
**20.03.91 Patentblatt 91/12**

(84) Benannte Vertragsstaaten:
**AT BE CH DE DK ES FR GB GR IT LI LU NL**



(71) Anmelder: **CIBA-GEIGY AG**
**Klybeckstrasse 141**
**CH-4002 Basel(CH)**

(72) Erfinder: **Kristiansen, Odd, Dr.**
**Delligrabenstrasse 7**
**CH-4313 Möhlin(CH)**
Erfinder: **Maienfisch, Peter, Dr.**
**Traugott Meyer-Strasse 5**
**CH-4147 Aesch(CH)**
Erfinder: **Gsell, Laurenz, Dr.**
**Maiengasse 56**
**CH-4056 Basel(CH)**


(54) **Guanidinderivate.**


(57) Neue Nitroguanidine der Formel I

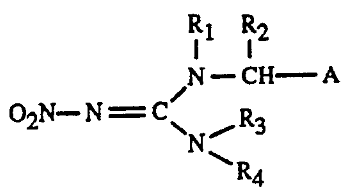

(I)

worin
$R_1$ für Wasserstoff, $C_1$-$C_4$-Alkyl oder $C_3$-$C_6$-Cycloalkyl,
$R_2$ für Wasserstoff oder $C_1$-$C_4$-Alkyl,
$R_3$ für Wasserstoff, $C_1$-$C_4$-Alkyl oder $C_3$-$C_6$-Cycloalkyl,
$R_4$ für Wasserstoff, $C_1$-$C_4$-Alkyl, $C_3$-$C_6$-Cycloalkyl oder ein Rest $-CHR_5-B$, oder
$R_3$ und $R_4$ gemeinsam für $-(CH_5)_4-$ oder $-(CH_2)_5-$,
$R_5$ für Wasserstoff oder $C_1$-$C_4$-Alkyl,
A für einen unsubstituierten oder ein- bis vierfach substituierten aromatischen oder nichtaromatischen, monocyclischen oder bicyclischen heterocyclischen Rest und
B für Phenyl, Cyanophenyl, Nitrophenyl, Halogenphenyl mit 1 bis 3 Halogenatomen oder jeweils unsubstituiertes oder substituiertes 5-Thiazolyl oder 3-Pyridyl stehen, sowie deren Salze können als Schädlingsbekämpfungsmittel eingesetzt werden. Vorzugsweise können Insekten und Arachniden bekämpft werden.


EP 0 418 199 A2

# GUANIDINDERIVATE

Die vorliegende Erfindung betrifft neue Derivate von Nitroguanidinen, Verfahren zu ihrer Herstellung, Schädlingsbekämpfungsmittel, welche diese Verbindungen enthalten, sowie ihre Verwendung bei der Kontrolle von Schädlingen.

Die erfindungsgemässen Nitroguanidinderivate entsprechen der Formel I

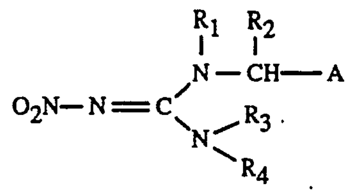

(I)

worin

$R_1$ für Wasserstoff, $C_1$-$C_4$-Alkyl oder $C_3$-$C_6$-Cycloalkyl,

$R_2$ für Wasserstoff oder $C_1$-$C_4$-Alkyl,

$R_3$ für Wasserstoff, $C_1$-$C_4$-Alkyl oder $C_3$-$C_6$-Cycloalkyl,

$R_4$ für Wasserstoff, $C_1$-$C_4$-Alkyl, $C_3$-$C_6$-Cycloalkyl oder ein Rest $-CHR_5-B$, oder

$R_3$ und $R_4$ gemeinsam fur-$(CH_2)_4$- oder -$(CH_2)_5$-,

$R_5$ für Wasserstoff oder $C_1$-$C_4$-Alkyl,

A für einen unsubstituierten oder ein- bis vierfach substituierten aromatischen oder nichtaromatischen, monocyclischen oder bicyclischen heterocyclischen Rest, wobei ein bis zwei Substituenten aus der Gruppe $C_1$-$C_3$-Halogenalkyl, Cyclopropyl, Halogencyclopropyl, $C_2$-$C_3$-Alkenyl, $C_2$-$C_3$-Alkinyl, $C_2$-$C_3$-Halogenalkenyl, $C_2$-$C_3$-Halogenalkinyl, $C_1$-$C_3$-Halogenalkoxy, $C_1$-$C_3$-Alkylthio, $C_1$-$C_3$-Halogenalkylthio, Allyloxy, Propargyloxy, Allylthio, Propargylthio, Halogenallyloxy, Halogenallylthio, Cyan und Nitro und ein bis vier Substituenten aus der Gruppe $C_1$-$C_3$-Alkyl, $C_1$-$C_3$-Alkoxy und Halogen ausgewählt sind, und

B für Phenyl, Cyanophenyl, Nitrophenyl, Halogenphenyl mit 1 bis 3 Halogenatomen, 3-Pyridyl, 5-Thiazolyl, durch ein bis zwei Substituenten aus der Gruppe $C_1$-$C_3$-Alkyl, $C_1$-$C_3$-Halogenalkyl, Cyclopropyl, Halogencyclopropyl, $C_2$-$C_3$-Alkenyl, $C_2$-$C_3$-Alkinyl, $C_1$-$C_3$-Alkoxy, $C_2$-$C_3$-Halogenalkenyl, $C_2C_3$-Halogenalkinyl, $C_1$-$C_3$-Halogenalkoxy, $C_1$-$C_3$-Alkylthio, $C_1$-$C_3$-Halogenalkylthio, Allyloxy, Propargyloxy, Allylthio, Propargylthio, Halogenallyloxy, Halogenallylthio, Halogen, Cyan und Nitro substituiertes 5-Thiazolyl; oder durch ein bis zwei Reste aus der Gruppe $C_1$-$C_3$-Halogenalkyl, Cyclopropyl, Halogencyclopropyl, $C_2$-$C_3$-Alkenyl, $C_2$-$C_3$-Alkinyl, $C_2$-$C_3$-Halogenalkenyl, $C_2$-$C_3$-Halogenalkinyl, $C_1$-$C_3$-Halogenalkoxy, $C_1$-$C_3$-Alkylthio, $C_1$-$C_3$-Halogenalkylthio, Allyloxy, Propargyloxy, Allylthio, Propargylthio, Halogenallyloxy, Halogenallylthio, Cyan und Nitro oder durch ein bis vier Reste aus der Gruppe $C_1$-$C_3$-Alkyl, $C_1$-$C_3$-Alkoxy oder Halogen substituiertes 3-Pyridyl stehen; sowie deren Salze mit anorganischen Säuren; unter Ausnahme der Verbindungen 1-Nitro-2-(pyrid-3-ylmethyl)-guanidin, 1-Nitro-2-(pyrid-2-ylmethyl)-guanidin, 1-Nitro-2-(pyrid-4-ylmethyl)-guanidin, 1-Nitro-2-(1-oxo-pyrid-3-ylmethyl)-guanidin und 1-(Benzimidazol-2-ylmethyl)-2-nitroguanidin.

Aus der Literatur sind heterocyclische Verbindungen, welche eine Nitroguanidinstruktur enthalten, aus der EP-A-192060 als Insektizide bekannt. Die biologischen Eigenschaften dieser Verbindungen vermögen jedoch bei der Schädlingsbekämpfung nicht voll zu befriedigen. Die aus der Definition der Formel I ausgeschlossenen Einzelverbindungen sind in der Literatur als Zwischenprodukte für pharmazeutisch aktive Verbindungen mit antileukemischen und antimikrobiellen Wirkungen beschrieben. Diese Verbindungen weisen ebenfalls die biologischen Eigenschaften der übrigen Substanzen der Formel I auf. Sie bilden daher bei der Verwendung und als Wirkstoff in agrochemischen Mitteln ebenfalls einen Bestandteil vorliegender Erfindung.

Die Verbindungen der erfindungsgemässen Formel I schliessen auch die Salze mit agrochemisch verträglichen anorganischen Säuren ein. Beispiele solcher Säuren sind Salzsäure, Bromwasserstoffsäure, Schwefelsäure, Phosphorsäure und Salpetersäure sowie Säuren mit gleichem Zentralatom und höherer oder niedrigerer Oxidationsstufen wie Perchlorsäure, salpettige Säure oder phosphorige Säure.

Die Verbindungen der Formel I können auch in den tautomeren Formen Ia oder Ib auftreten, wenn mindestens einer der Reste $R_1$, $R_3$ oder $R_4$ für Wasserstoff steht.

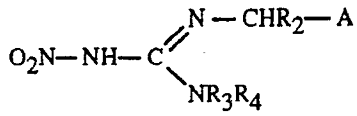

(Ia)

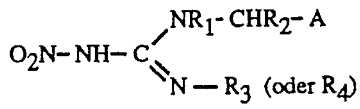

(Ib)

Die erfindungsgemässe Formel I ist hierin so zu verstehen, dass die Formen Ia und Ib in der Schreibweise der Formel I eingeschlossen sind.

Die in der Definition des heterocyclischen Restes A eingeschlossenen Ringsysteme enthalten als Ringglied mindestens ein Heteroatom, das heisst mindestens eines der den zugrundeliegenden ringförmigen Grundkörper bildenden Atome ist von Kohlenstoff verschieden. Grundsätzlich sind alle Atome des periodischen Systems der Elemente befähigt, als Ringglieder zu fungieren, sofern sie mindestens zwei kovalente Bindungen bilden können. Der heterocyclische Rest ist dabei bevorzugt ungesättigt und über ein Kohlenstoffatom als Ringglied an den Grundkörper des Nitroguanidins der Formel I gebunden. Ungesättigte Ringsysteme der Definition A enthalten eine oder mehrere Doppelbindungen, vorzugsweise sind derartige Ringsysteme mehrfach ungesättigt und weisen im allgemeinen einen aromatischen Charakter auf. Bevorzugt sind solche Ringsysteme, welche als Heteroatom mindestens ein Stickstoffatom enthalten. Üblicherweise enthalten solche Ringe der Definition A ein bis drei Heteroatome aus der Reihe Sauerstoff, Schwefel und Stickstoff, wobei jeweils höchstens ein Sauerstoff- oder Schwefelatom enthalten ist. Bevorzugt sind Ringsysteme unter der Definition von A, worin der heterocyclische Rest A ein bis drei Heteroatome aus der Reihe Sauerstoff, Schwefel und Stickstoff enthält, wovon stets ein Heteoatom Stickstoff ist und höchstens ein Sauerstoffatom oder Schwefelatom enthalten ist. Insbesondere finden sich Beispiele für erfindungsgemässe Heterocyclen der Definition A in der Gruppe von Grundkörpern der folgenden Strukturen:

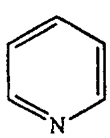 , 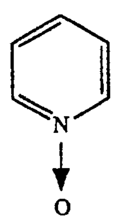 , 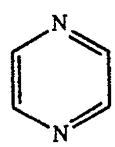 , 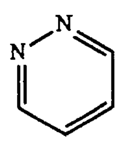 , 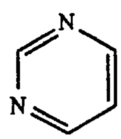 , 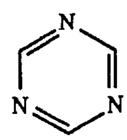 , 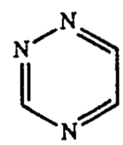 ,

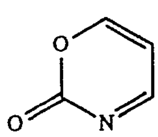 , 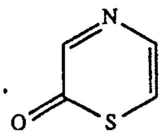 , 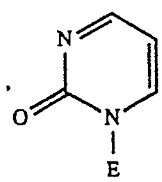 , 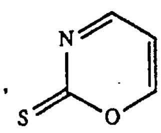 , 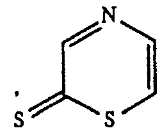 , 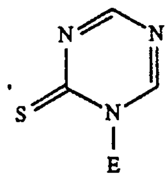 ,

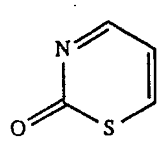 , 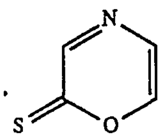 , 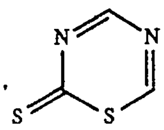 , 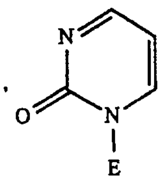 , 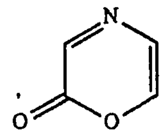 ,

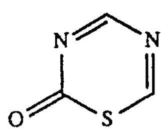 , 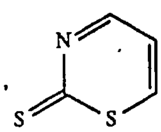 , 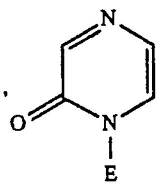 , 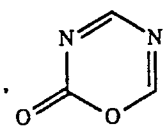 , 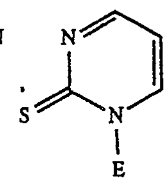 , 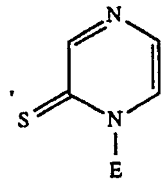 ,

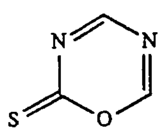 , 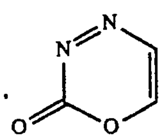 , 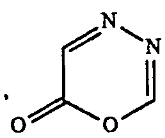 , 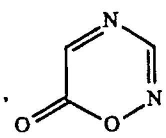 , 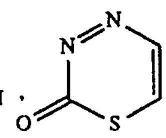 ,

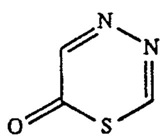 , 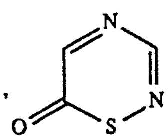 , 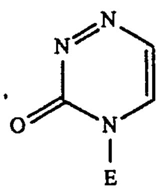 , 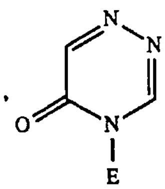 , 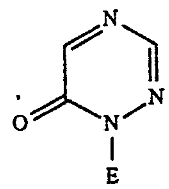 ,

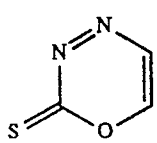 , 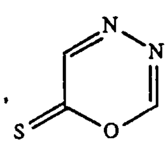 , 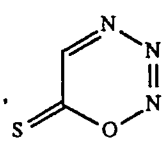 , 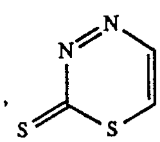 , 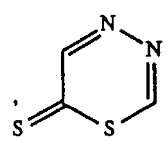 ,

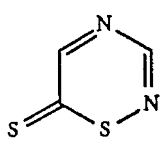 , 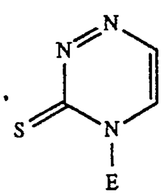 , 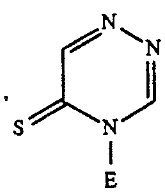 , 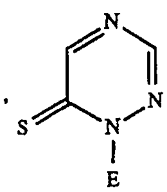 , 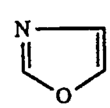 ,

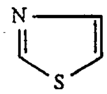 , 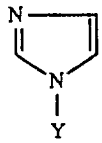 , 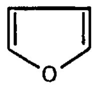 , 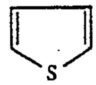 ,

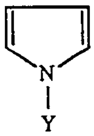 , 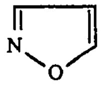 , 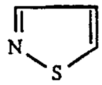 , 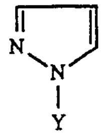 ,

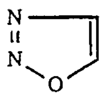 , 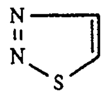 , 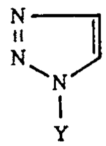 , 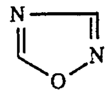 , 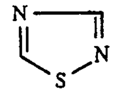 ,

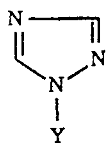 , 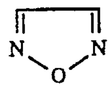 , 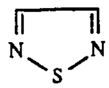 , 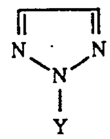 , 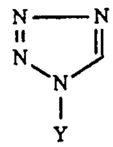 ,

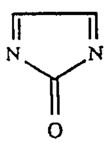 , 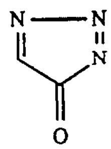 , 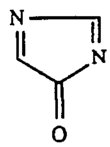 , 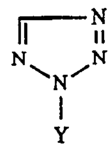 , 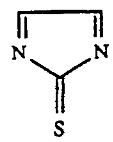

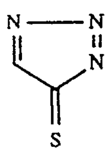 , 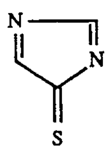 , 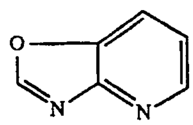 , 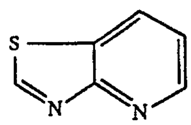 , 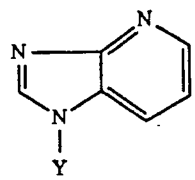 ,

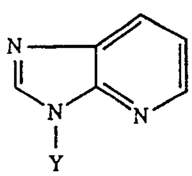 , 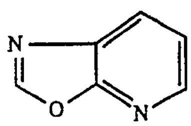 , 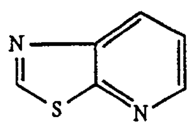 , 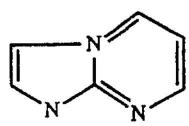 ,

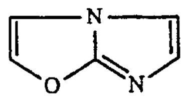 , 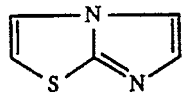 , 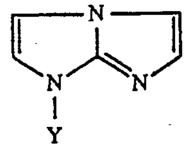 ,

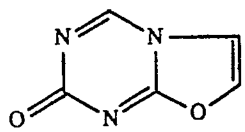 , 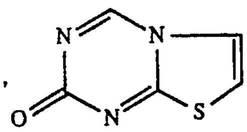 , 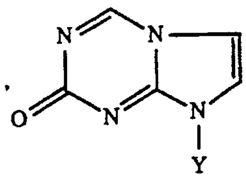 , 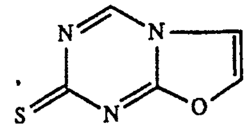 ,

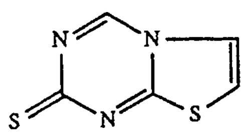 und 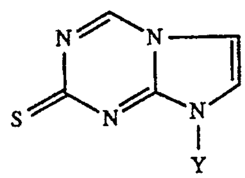 .

In den obigen Formeln steht
E für $C_1$-$C_3$-Alkyl und
Y für Wasserstoff, $C_1$-$C_3$-Alkyl oder Cyclopropyl.

Die beispielhaft aufgezählten Heterocyclen A können unsubstituiert sein oder je nach Substitutionsmöglichkeiten des Ringsystems bis zu vier Substituenten tragen, wie sie unter Formel I angegeben sind. Bevorzugt sind diese Heterocyclen in unsubstituierter Form über ein Kohlenstoffatom an den Guanidinkörper gebunden, oder sie tragen ein bis drei Substituenten aus der Gruppe Halogen, $C_1$-$C_3$-Alkyl, $C_1$-$C_3$-Halogenalkyl, $C_1$-$C_3$-Halogenalkoxy oder $C_1$-$C_3$-Alkoxy. Ganz besonders bevorzugte Heterocyclen A sind Pyridylreste oder Thiazolylreste, wie zum Beispiel 3-Pyridyl, 2-Halogenpyrid-5-yl, 2,3-Dihalogenpyrid-5-yl, 2-Halogenthiazol-4-yl, 1-Oxopyrid-3-yl, 1-Oxo-2-halogenpyrid-5-yl, 1-Oxo-2,3-dihalogenpyrid-5-yl.

In der Definition der erfindungsgemässen Formel I sollen die einzelnen generischen Begriffe wie folgt verstanden werden:

Bei den als Substituenten in Betracht kommenden Halogenatomen handelt es sich sowohl um Fluor und Chlor als auch um Brom und Jod, wobei Fluor, Chlor und Brom bevorzugt sind. Halogen ist dabei als selbständiger Substituent oder als Teil eines Substituenten zu verstehen wie im Halogenalkyl, Halogenalkylthio, Halogenalkoxy, Halogencycloalkyl, Halogenalkenyl, Halogenalkinyl, Halogenallyloxy oder Halogenallylthio. Die als Substituenten in Betracht kommenden Alkyl-, Alkylthio-, Alkenyl-, Alkinyl- und Alkoxyreste können geradkettig oder verzweigt sein. Als Beispiele solcher Alkyle seien Methyl, Aethyl, Propyl, Isopropyl, Butyl, i-Butyl, sek.-Butyl oder tert.-Butyl genannt. Als geeignete Alkoxyreste seien unter anderem genannt: Methoxy, Aethoxy, Propoxy, Isopropoxy oder Butoxy und ihre Isomeren. Alkylthio steht beispielsweise für Methylthio, Aethylthio, Isopropylthio, Propylthio oder die isomeren Butylthio. Sind die als Substituenten in Betracht kommenden Alkyl-, Alkoxy-, Alkenyl-, Alkinyl- oder Cycloalkylgruppen durch Halogen substituiert, so können sie nur teilweise oder auch perhalogeniert sein. Dabei gelten für Halogen, Alkyl und Alkoxy die oben gegebenen Definitionen. Beispiele der Alkylelemente dieser Gruppen sind das ein- bis dreifach durch Fluor, Chlor und/oder Brom substituierte Methyl wie beispielsweise $CHF_2$ oder $CF_3$; das ein- bis fünffach durch Fluor, Chlor und/oder Brom substituierie Aethyl wie zum Beispiel $CH_2CF_3$, $CF_2CF_3$, $CF_2CCl_3$, $CF_2CHCl_2$, $CF_2CHF_2$, $CF_2CFCl_2$, $CF_2CHBr_2$, $CF_2CHClF$, $CF_2CHBrF$ oder $CClFCHClF$; das ein- bis siebenfach durch Fluor, Chlor und/oder Brom substituierte Propyl oder Isopropyl wie beispielsweise $CH_2CHBrCH_2Br$, $CF_2CHFCF_3$, $CH_2CF_2CF_3$ oder $CH(CF_3)_2$; das ein- bis neunfach durch Fluor, Chlor und/oder Brom substituierte Butyl oder eines seiner Isomeren wie zum Beispiel $CF(CF_3)CHFCF_3$ oder $CH_2$-$(CF_2)_2CF_3$; 2-Chlorcyclopropyl oder 2,2-Difluorcyclopropyl; 2,2-Difluorvinyl, 2,2-Dichlorvinyl, 2-Chloralkyl, 2,3-Dichlorvinyl oder 2,3-Dibromvinyl.

Sind die definierten Alkyl-, Alkoxy- oder Cycloalkylgruppen durch andere Substituenten substituiert, so können sie ein- oder mehrfach durch den gleichen oder verschiedene der aufgezählten Substituenten substituiert sein. Vorzugsweise sind in den substituierten Gruppen ein oder zwei weitere Substituenten vorhanden. Bei den als Substituenten in Betracht kommenden Cycloalkylresten handelt es sich beispielsweise um Cyclopropyl, Cyclobutyl, Cyclopentyl oder Cyclohexyl. Alkenyl- und Alkinylgruppen enthalten eine

ungesättigte Kohlenstoff-Kohlenstoff-Bindung. Typische Vertreter sind Allyl, Methallyl oder Propargyl, aber auch Vinyl und Äthinyl. Die Doppel- oder Dreifachbindungen in Allyloxy, Propargyloxy, Allylthio oder Propargylthio sind von der Verknüpfungsstelle zum Heteroatom (O oder S) vorzugsweise durch ein gesättigtes Kohlenstoffatom getrennt.

Unter den Verbindungen der Formel I sind solche Untergruppen herauszuheben, in denen entweder

a) $R_1$ und $R_3$ unabhängig voneinander für Wasserstoff, Methyl, Äthyl oder Cyclopropyl stehen, oder

b) $R_2$ Wasserstoff bedeutet, oder

c) $R_4$ Wasserstoff oder Methyl bedeutet.

Für den Fall, dass $R_4$ für die Gruppe -$CHR_5$-B steht, sind diejenigen Verbindungen bevorzugt, worin $R_5$ Wasserstoff bedeutet. Die bevorzugte Ausführungsform für B sind Phenyl, Pyridyl oder Thiazolyl, welche jeweils unsubstituiert oder durch ein bis drei Reste aus der Gruppe Halogen, $C_1$-$C_3$-Alkyl, $C_1$-$C_3$-Halogenalkyl, $C_1$-$C_3$-Halogenalkoxy oder $C_1$-$C_3$-Alkoxy substituiert sind.

Eine besonders bevorzugte Gruppe von Verbindungen der Formel I ist dadurch gekennzeichnet, dass $R_1$ und $R_3$ unabhängig voneinander Wasserstoff, Methyl, Äthyl oder Cyclopropyl, $R_2$ Wasserstoff, $R_4$ Wasserstoff oder Methyl und A Pyridyl, 1-Oxopyridyl, Thiazolyl oder jeweils durch ein bis drei Substituenten aus der Gruppe Halogen, $C_1$-$C_3$-Alkyl, $C_1$-$C_3$-Halogenalkyl, $C_1$-$C_3$-Halogenalkoxy oder $C_1$-$C_3$-Alkoxy substituiertes Pyridyl, 1-Oxopyridyl oder Thiazolyl bedeuten.

Als bevorzugte Einzelverbindungen der Formel I sind zu nennen:

1-(2-Chlorpyrid-5-ylmethyl)-1-methyl-2-nitroguanidin,
1-(2-Chlorpyrid-5-ylmethyl)-2-nitroguanidin,
1-Methyl-2-nitro-3-(pyrid-3-ylmethyl)-guanidin,
1-Methyl-2-nitro-1-(pyrid-3-ylmethyl)-guanidin,
1,2-Dimethyl-3-nitro-1-(pyrid-3-ylmethyl)-guanidin,
1-(2-Chlorpyrid-5-ylmethyl)-1,2-dimethyl-3-nitroguanidin,
1-(2-Chlorthiazol-5-ylmethyl)-1-methyl-2-nitroguanidin,
1-(2-C hlorpyrid-5-ylmethyl)-2,2-dimethyl-3-nitroguanidin,
1-(2-Chlorpyrid-5-ylmethyl)2-methyl-3-nitroguanidin,
1,1-Dimethyl-2-nitro-3-(pyrid-3-ylmethyl)-guanidin,
1-Aethyl-2-nitro-3-(pyrid-3-ylmethyl)-guanidin,
1-Aethyl-2(2-chlorpyrid-5-ylmethyl)-3-nitroguanidin,
1-Aethyl-1-methyl-2-(2-chlorpyrid-5-ylmethyl)-3-nitroguanidin
1-(2-Chlorthiazol-5-ylmethyl)-2,2-dimethyl-3-nitroguanidin,
1-(2,3-Dichlorpyrid-5-ylmethyl)-2-methyl-3-nitroguanidin,
1-Aethyl-2-(2,3-dichlorpyrid-5-ylmethyl)-3-nitroguanidin,
1-(2,3-Dichlorpyrid-5-ylmethyl)-1-methyl-2-nitroguanidin,
1-Aethyl-1-(2,3-dichlorpyrid-5-ylmethyl)-2-nitroguanidin,
1-(2-Chlor-1-oxopyrid-5-ylmethyl)-1-methyl-2-nitroguanidin,
1-Aethyl-1-(2-chlor-1-oxopyrid-5-ylmethyl)-2-nitroguanidin,
1-(2-Chlor-1-oxopyrid-5-ylmethyl)-2-methyl-3-nitroguanidin,
1-Aethyl-1-(2-chlor-1-oxopyrid-5-ylmethyl)-3-nitroguanidin,
1-(2-Chlorthiazol-5-ylmethyl)-2-methyl-3-nitroguanidin,
1-(2-Chlorthiazol-5-ylmethyl)-2-nitroguanidin und
1-Aethyl-2-(2-chlorthiazol-5-ylmethyl)-3-nitroguanidin.

Die eifindungsgemässen Verbindungen der Formel I können in Analogie zu bekannten Verfahren hergestellt werden. Beispielsweise erhält man die Verbindung der Formel I, indem man entweder

a) ein Amin der Formel II

H-$NR_1$-$CHR_2$-A (II)

mit einem Nitroisothioharnstoff der Formel III

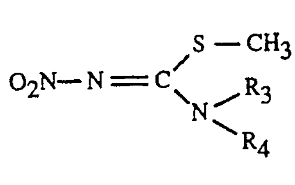

(III),

worin $R_1$, $R_2$, $R_3$, $R_4$ und A die unter Formel I gegebenen Bedeutungen haben, umsetzt, oder

b) eine Verbindung der Formel IV

Hal-$CHR_2$-A (IV)

in Gegenwart einer Base mit einem Nitroguanidinderivat der Formel V

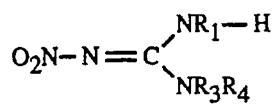

(V),

worin $R_1$, $R_2$, $R_3$, $R_4$ und A die unter Formel I gegebenen Bedeutungen haben und Hal für Halogen, vorzugsweise Chlor oder Brom, steht, umsetzt.

Die Verbindungen der Formel I, worin $R_3$ und $R_4$ Wasserstoff bedeuten, kann man auch dadurch erhalten, dass man ein Amin der Formel II

H-$NR_1$-$CHR_2$-A (II),

worin $R_1$, $R_2$ und A die unter Formel I gegebenen Bedeutungen haben, entweder mit 1-Methyl-1-nitroso-2-nitroguanidin der Formel VI

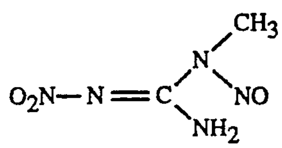

(VI)

oder mit Nitroguanidin der Formel VII

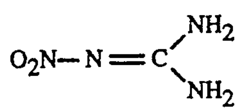

(VII)

umsetzt.

Die Durchführung der Variante a) des erfindungsgemässen Verfahrens (II + III → I) erfolgt mit Vorteil in einem inerten Lösungsmittel bei Temperaturen zwischen 0° C und +150° C, insbesondere zwischen +40° C und +120° C. Als Lösungsmittel eignen sich in besonderer Weise Alkohole wie Methanol, Äthanol oder Isopropanol. Die Variante b) (IV + V → I) wird mit Vorteil in einem inerten polaren aprotischen Lösungsmittel wie Acetonitril oder Dimethylformamid bei Temperaturen zwischen +50° C und der Siedetemperatur des Reaktionsgemisches durchgeführt. Als Basen eignen sich Carbonate wie Kaliumcarbonat. Auch Hydride wie Natriumhydrid können als Basen eingesetzt werden. Beim Einsatz von Natriumhydrid in Acetonitril oder Dimethylformamid kann die Reaktionstemperatur auf +10° C bis +25° C herabgesetzt werden, ohne dass sich dadurch die Reaktionszeit wesentlich verlängert. Die Umsetzung von II mit VI wird mit Vorteil in wässrigen, alkoholischen Lösungen bei Temperaturen zwischen 0° C und +25° C durchgeführt. Bei dieser Umsetzung entsteht als Nebenprodukt N-Nitrosomethylamin. Die Umsetzung von II mit VII wird üblicherweise in wässrigem Medium bei Temperaturen zwischen +40° C und +100° C, vorzugsweise zwischen +60° C und +80° C durchgeführt.

Aus Verbindungen der Unterformel Ia

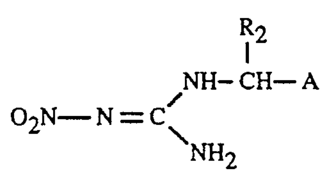

(Ia)

worin $R_2$ und A die unter Formel I gegebenen Bedeutungen haben, können durch Alkylierungsreaktion in Gegenwart einer Base unter Verwendung der Alkylierungsreagenzien

$R_1$-Hal,
$R_3$-Hal oder
$R_4$-Hal
worin $R_1$, $R_3$ und $R_4$ die unter Formel I gegebenen Bedeutungen haben, welche nicht Wasserstoff sind, und Hal für Halogen, vorzugsweise Chlor oder Brom steht, diejenigen Guanidinderivate der Formel I hergestellt werden, in welchen $R_1$, $R_3$ und $R_4$ diejenigen Bedeutungen haben, welche nicht Wasserstoff sind.

Da bekanntlich Alkylierungsreaktionen des obigen Typs kaum regiospezifisch verlaufen, eignet sich dieser Alkylierungsschritt vorzugsweise zur Herstellung tertiärer Aminogruppen, also insbesondere für die Fälle, in denen gleichzeitig alle drei verbliebenen Wasserstoffatome an den Guanidin-Stickstoffatomen durch gleichartige Reste $R_1$, $R_3$ und $R_4$ substituiert werden sollen.

Andererseits können durch Alkylierungen des obigen Typs auf einfache Weise solche Guanidinderivate der Formel I erhalten werden, worin sämtliche Reste $R_1$, $R_3$ und $R_4$ eine von Wasserstoff verschiedene Bedeutung haben, wenn man jeweils nur noch einen dieser Reste anstelle eines Wasserstoffatoms einführen möchte. Bei genügend grosser sterischer Hinderung durch entweder die vorhandenen Reste im Guanidingerüst wie $-CHR_2-A$ oder die einzuführenden Reste $R_1$, $R_3$ oder $R_4$ kann die Alkylierungsreaktion dieses Typs dennoch zur Herstellung sekundärer Guanidin-Aminogruppen angewendet werden. Werden die Verbindungen der Formel Ia nur mit einem oder zwei Aequivalenten Alkylierungsreagenz umgesetzt, entstehen sowohl Isomerengemische als auch Alkylierungsprodukte mit unterschiedlichem Alkylierungsgrad. Diese Gemische können jedoch mit üblichen Trennungsmethoden in ihre Einzelbestandteile aufgetrennt werden, so dass unter anderen auch das gewünschte Produkt ausgewählt werden kann.

Als Reaktionsbedingungen wählt man für die genannten Alkylierungsreaktionen eine Temperatur zwischen 0°C und +150°C, vorzugsweise zwischen +10°C und +50°C. Als Basen eignen sich Alkali- und Erdalkalihydride wie Natriumhydrid oder Calciumhydrid oder Carbonate wie Natriumcarbonat oder Kaliumcarbonat. Mit Vorteil führt man die Alkylierungsreaktion in einem polaren, aprotischen Lösungsmittel wie Acetonitril oder Dimethylformamid durch.

Die Zwischenprodukte der Formeln II, III, IV, V, VI und VII sind bekannt oder können in Analogie zu bekannten Verfahren hergestellt werden.

Es wurde nun gefunden, dass die erfindungsgemässen Verbindungen der Formel I bei günstiger Warmblüter-, Fisch- und Pflanzenverträglichkeit wertvolle Wirkstoffe in der Schädlingsbekämpfung sind. Insbesondere betrifft die Anwendung der erfindungsgemässen Wirkstoffe Insekten und Spinnentiere, die in Nutz- und Zierpflanzen in der Landwirtschaft, insbesondere in Baumwoll-, Gemüse- und Obstpflanzungen, im Forst, im Vorrats- und Materialschutz sowie im Hygienesektor insbesondere an Haus- und Nutztieren vorkommen. Sie sind gegen alle oder einzelne Entwicklungsstadien von normal sensiblen aber auch resistenten Arten wirksam. Dabei kann sich ihre Wirkung in unmittelbarer Abtötung der Schädlinge oder erst nach einiger Zeit, beispielsweise bei einer Häutung, oder in einer verminderten Eiablage und/oder Schlupfrate zeigen. Zu den oben erwähnten Schädlingen gehören:
aus der Ordnung Lepidoptera zum Beispiel
Acleris spp., Adoxophyes spp., Aegeria spp., Agrotis spp., Alabama argillaceae, Amylois spp., Anticarsia gemmatalis, Archips spp., Argyrotaenia spp., Autographa spp., Busseola fusca, Cadra cautella, Carposina nipponensis, Chilo spp., Choristoneura spp., Clysia ambiguella, Cnaphalocrocis spp., Cnephasia spp., Cochylis spp., Coleophora spp., Crocidolomia binotalis, Cryptophlebia leucotreta, Cydia spp., Diatraea spp., Diparopsis castanea, Earias spp., Ephestia spp., Eucosma spp., Eupoecilia ambiguella, Euproctis spp., Euxoa spp., Grapholita spp., Hedya nubiferana, Heliothis spp., Hellula undalis, Hyphantria cunea, Keiferia lycopersicella, Leucoptera scitella, Lithocollethis spp., Lobesia botrana, Lymantria spp., Lyonetia spp., Malacosoma spp., Mamestra brassicae, Manduca sexta, Operophtera spp., Ostrinia nubilalis, Pammene spp., Pandemis spp., Panolis flammea, Pectinophora gossypiella, Phthorimaea operculella, Pieris rapae, Pieris spp., Plutella xylostella, Prays spp., Scirpophaga spp., Sesamia spp., Sparganothis spp., Spodoptera spp., Synanthedon spp., Thaumetopoea spp., Tortrix spp., Trichoplusia ni und Yponomeuta spp.;
aus der Ordnung Coleoptera zum Beispiel
Agriotes spp., Anthonomus spp., Atomaria linearis, Chaetocnema tibialis, Cosmopolites spp., Curculio spp., Dermestes spp., Diabrotica spp., Epilachna spp., Eremnus spp., Leptinotarsa decemlineata, Lissorhoptrus spp. Melolontha spp., Orycaephilus spp., Otiorhynchus spp., Phlyctinus spp., Popillia spp., Psylliodes spp., Rhizopertha spp., Scarabeidae, Sitophilus spp., Sitotroga spp., Tenebrio spp., Tribolium spp. und Trogoderma spp.; aus der Ordnung der Orthoptera zum Beispiel Blatta spp., Blattella spp., Gryllotalpa spp., Leucophaea maderae, Locusta spp., Periplaneta spp. und Schistocerca spp.;
aus der Ordnung der Isoptera zum Beispiel
Reticulitermes spp.; aus der Ordnung der Psocoptera zum Beispiel Liposcelis spp.; aus der Ordnung der Anoplura zum Beispiel Haematopinus spp., Linognathus spp. Pediculus spp., Pemphigus spp. und Phylloxera spp.; aus der Ordnung der Mallophaga zum Beispiel Damalinea spp. und Trichodectes spp.;
aus der Ordnung der Thysanoptera zum Beispiel
Frankliniella spp., Hercinothrips spp., Taeniothrips spp., Thrips palmi, Thrips tabaci und Scirtothrips aurantii;
aus der Ordnung der Heteroptera zum Beispiel
Cimex spp., Distantiella theobroma, Dysdercus spp., Euchistus spp. Eurygaster spp. Leptocorisa spp., Nezara spp., Plesma spp., Rhodnius spp., Sahlbergella singularis, Scotinophara spp. und Triatoma spp.;
aus der Ordnung der Homoptera zum Beispiel
Aleurothrixus floccosus, Aleyrodes brassicae, Aonidiella spp., Aphididae, Aphis spp., Aspidiotus spp., Bemisia tabaci, Ceroplaster spp., Chrysomphalus aonidium, Chrysomphalus dictyospermi, Coccus hesperidum, Empoasca spp., Eriosoma larigerum, Erythroneura spp., Gascardia spp., Laodelphax spp., Lecanium corni, Lepidosaphes spp., Macrosiphus spp., Myzus spp., Nephotettix spp., Nilaparvata spp., Paratoria spp., Pemphigus spp., Planococcus spp., Pseudaulacaspis spp., Pseudococcus spp., Psylla spp., Pulvinaria aethiopica, Quadraspidiotus spp., Rhopalosiphum spp., Saissetia spp., Scaphoideus spp., Schizaphis spp., Sitobion spp., Trialeurodes vaporariorum, Trioza erytreae und Unaspis citri;
aus der Ordnung der Hymenoptera zum Beispiel
Acromyrmex, Atta spp., Cephus spp., Diprion spp., Diprionidae, Gilpinia polytoma, Hoplocampa spp., Lasius spp., Monomorium pharaonis, Neodiprion spp., Solenopsis spp. und Vespa spp.;
aus der Ordnung der Diptera zum Beispiel
Aedes spp., Antherigona soccata, Bibio hortulanus, Calliphora erythrocephala, Ceratitis spp., Chrysomyia spp., Culex spp., Cuterebra spp., Dacus spp., Drosophila melanogaster, Fannia spp., Gastrophilus spp., Glossina spp., Hypoderma spp., Hyppobosca spp., Liriomyza spp., Lucilia spp., Melanagromyza spp., Musca spp., Oestrus spp., Orseolia spp. Oscinella frit, Pegomyia hyoscyami, Phorbia spp., Rhagoletis pomonella, Sciara spp., Stomoxys spp., Tabanus spp., Tannia spp. und Tipula spp.;
aus der Ordnung der Siphonaptera z.B.
Ceratophyllus spp., Xenopsylla cheopis,
aus der Ordnung der Acarina zum Beispiel
Acarus siro, Aceria sheldoni, Aculus schlechtendali, Amblyomma spp., Argas spp., Boophilus spp., Brevipalpus spp., Bryobia praetiosa, Calipitrimerus spp., Chorioptes spp., Dermanyssus gallinae, Eotetranychus carpini, Eriophyes spp., Hyalomma spp., Ixodes spp., Olygonychus pratensis, Ornithodoros spp., Panonychus spp., Phyllocoptruta oleivora, Polyphagotarsonemus latus, Psoroptes spp., Rhipicephalus spp., Rhizoglyphus spp.,
Sarcoptes spp., Tarsonemus spp. und Tetranychus spp.; und
aus der Ordnung der Thysanura zum Beispiel
Lepisma saccharina.

Die gute pestizide Wirkung der erfindungsgemässen Verbindungen der Formel I entspricht einer Abtötungsrate (Mortalität) von mindestens 50-60 % der erwähnten Schädlinge.

Die Wirkung der eifindungsgemässen Verbindungen und der sie enthaltenden Mittel lässt sich durch Zusatz von anderen Insektiziden und/oder Akariziden wesentlich verbreitern und an gegebene Umstände anpassen. Als Zusätze kommen zum Beispiel Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die Verbindungen der Formel I werden in unveränderter Form oder vorzugsweise zusammen mit den in der Formulierungstechnik üblichen Hilfsmitteln eingesetzt und können daher beispielsweise zu emulgierbaren Konzentraten, direkt versprüh- oder verdünnbaren Lösungen, verdünnten Emulsionen, Spritzpulvern, löslichen Pulvern, Stäubemitteln, Granulaten, auch Verkapselungen in polymeren Stoffen in bekannter Weise verarbeitet werden. Die Anwendungsverfähren, wie Versprühen, Vernebeln, Verstäuben, Verstreuen oder Giessen, werden ebenso wie die Mittel den angestrebten Zielen und den gegebenen Verhältnissen entsprechend gewählt. Ferner eignen sich die Verbindungen der Formel I auch für den Einsatz bei der Behandlung von Saatgut. Dabei kann sowohl das Saatgut vor dem Säen mit dem Wirkstoff oder einer den Wirkstoff enthaltenden Formulierung behandelt oder gebeizt werden, als auch der Wirkstoff beim Säen in die Saatfurche appliziert werden.

Die Formulierung, das heisst die den Wirkstoff der Formel I, beziehungsweise Kombinationen dieser Wirkstoffe mit anderen Insektiziden oder Akariziden, und gegebenenfalls einen festen oder flüssigen Zusatzstoff enthaltenden Mittel, Zubereitungen oder Zusammensetzungen, werden in bekannter Weise hergestellt, zum Beispiel durch inniges Vermischen und/oder Vermahlen der Wirkstoffe mit Streckmitteln, wie beispielsweise mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen

$C_8$ bis $C_{12}$ von Alkylbenzolen wie Xylolgemische oder alkylierte Naphthaline, aliphatische oder cycloaliphatische Kohlenwasserstoffe wie Cyclohexan, Paraffine oder Tetrahydronaphthalin, Alkohole wie Aethanol, Propanol oder Butanol, und Glykole sowie deren Aether und Ester wie Propylenglykol, Dipropylenglykoläther, Aethylenglykol, Aethylenglykolmonomethyl- oder -äthyläther, Ketone wie Cyclohexanon, Isophoron oder Diacetanolalkohol, starke polare Lösungsmittel wie N-Methyl-2-pyrrolidon, Dimethylsulfoxid oder Dimethylformamid oder Wasser, Pflanzenöle wie Raps-, Rizinus-, Kokosnuss- oder Sojaöl; gegebenenfalls auch Silikonöle.

Als feste Trägerstoffe, beispielsweise für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen wie Bimsstein, Ziegelbruch, Sepiolit oder Bentonit, als nicht sorptive Trägermaterialien Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formel I oder der Kombinationen dieser Wirkstoffe mit andern Insektiziden oder Akariziden nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sogenannte wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$) wie die Natrium- oder Kalium-Salze der Oel- oder Stearinsäure, oder von natürlichen Fettsäuregemischen, die beispielsweise aus Kokosnuss- oder Tallöl gewonnen werden können. Ferner sind als Tenside auch die Fettsäuremethyl-taurin-salze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, beispielsweise das Natrium- oder Calcium-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind zum Beispiel die Natrium-, Calcium- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie zum Beispiel Salze des Phosphorsäureesters eines p-Nonylphenol-(4-14)- Aethylenoxid-Adduktes oder Phospholipide in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykol-Einheiten.

Als Beispiele nichtionischer Tenside seien Nonylphenolpolyäthoxyäthanole, Ricinusölpolyglykoläther, Polypropylen-Polyäthylenoxid-Addukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitan-trioleat in Betracht.

Bei den kationischen Tensiden handelt es sich vor allem um quarternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, beispielsweise das Stearyltrimethylammoniumchlorid oder das Benzyl-di-(2-chloräthyl)-äthylammoniumbromid.

Die in der Formulierungstechnik gebräuchlichen Tenside sind beispielsweise in folgenden Publikationen beschrieben:

"Mc Cutcheon's Detergents and Emulsifiers Annual", Mc Publishing Corp., Glen Rock, NJ, USA, 1988", H. Stache, "Tensid-Taschenbuch", 2. Aufl., C. Hanser Verlag München, Wien 1981.

M. and J. Ash. "Encyclopedia of Surfactants", Vol. I-III, Chemical Publishing Co., New York, 1980-1981.

Die pestiziden Zubereitungen enthalten in der Regel 0,1 bis 99 %, insbesondere 0,1 bis 95 % Wirkstoff der Formel I oder Kombinationen dieses Wirkstoffs mit andern Insektiziden oder Akariziden, 1 bis 99,9 % eines festen oder flüssigen Zusatzstoffes und 0 bis 25 %, insbesondere 0,1 bis 25 %, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen. Typische Anwendungskonzentrationen liegen zwischen 0,1 und 1000 ppm, vorzugsweise zwischen 0,1 und 500 ppm. Die Aufwandmengen pro Hektar betragen im allgemeinen 1 bis 1000g Wirkstoff pro Hektar, vorzugsweise 25 bis 500 g/ha.

Insbesondere setzen sich bevorzugte Formulierungen folgendermassen zusammen:
(% = Gewichtsprozent)

Emulgierbare Konzentrate:
Aktiver Wirkstoff: 1 bis 90 %, bevorzugt 5 bis 20 %
oberflächenaktives Mittel: 1 bis 30 %, vorzugsweise 10 bis 20 %
flüssiges Trägermittel: 5 bis 94 %, vorzugsweise 70 bis 85 %

Stäube:
Aktiver Wirkstoff: 0,1 bis 10 %, vorzugsweise 0,1 bis 1 %
festes Trägermittel: 99,9 bis 90 %, vorzugsweise 99,9 bis 99 %

Suspension-Konzentrate:
Aktiver Wirkstoff: 5 bis 75 %, vorzugsweise 10 bis 50 %
Wasser: 94 bis 24 %, vorzugsweise 88 bis 30 %
oberflächenaktives Mittel: 1 bis 40 %, vorzugsweise 2 bis 30 %

Benetzbare Pulver:
Aktiver Wirkstoff: 0,5 bis 90 %, vorzugsweise 1 bis 80 %
oberflächenaktives Mittel: 0,5 bis 20 %, vorzugsweise 1 bis 15 %
festes Trägermaterial: 5 bis 95 %, vorzugsweise 15 bis 90 %

Granulate:
Aktiver Wirkstoff: 0,5 bis 30 %, vorzugsweise 3 bis 15 %
festes Trägermittel: 99,5 bis 70 %, vorzugsweise 97 bis 85 %

Die Mittel können auch weitere Zusätze wie Stabilisatoren, z.B. gegebenenfalls epoxidierte Pflanzenöle (epoxidiertes Kokosnussöl, Rapsöl oder Sojaöl), Entschäumer, z.B. Silikonöl, Konservierungsmittel, Viskositätsregulatoren, Bindemittel, Haftmittel sowie Dünger oder andere Wirkstoffe zur Erzielung spezieller Effekte enthalten.

Die folgenden Beispiele dienen der Erläuterung der Erfindung. Sie schränken die Erfindung nicht ein.

Herstellungsbeispiele

Beispiel H1: 1-(2-Chlorpyrid-5-ylmethyl)-1-methyl-2-nitroguanidin

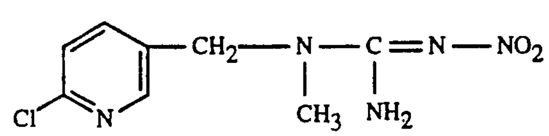

Eine Mischung von 3,2 g N-Methyl-(2-chlorpyrid-5-yl)-methylamin, 2,7 g S-Methyl-N-nitroisothioharnstoff, 0,2 g Kaliumhydrogensulfat und 75 mi Äthanol wird für 4,5 Stunden zum Rückfluss erhitzt. Die Reaktionsmischung wird heiss filtriert. Aus dem Filtrat kristallisiert beim Abkühlen auf 0° C das Rohprodukt aus. Durch Umkristallisieren aus Äthanol erhält man reines 1-(2-Chlorpyrid-5-ylmethyl)-1-methyl-2-nitroguanidin in Form eines farblosen Kristallisats mit einem Schmelzpunkt von 163-165° C.

Beispiel H2: 1-(2-Chlorpyrid-5-ylmethyl)-2-nitroguanidin

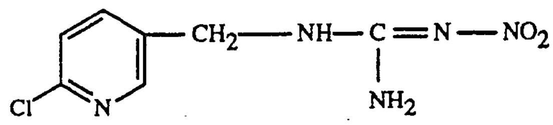

Eine Suspension von 4,0 g Nitroguanidin und 5,4 g (2-Chlorpyrid-5-yl)-methylamin in 80 mi Wasser wird für 3 Stunden auf +80°C erwärmt. Nach Abkühlen des Reaktionsgemisches auf +20°C wird innerhalb von 16 Stunden das Produkt in Form eines farblosen Kristallisats ausgeschieden. Das Kristallpulver wird abgetrennt, mit Methanol gewaschen und getrocknet. Man erhält so 2,0g 1-(2-Chlorpyrid-5-ylmethyl)-2-nitroguanidin mit einem Schmelzpunkt von 195- 197°C.

Beispiel H3: 1-(2-Chlorpyrid-5-ylmethyl)-2-(4-chlorbenzyl)-1-methyl-3-nitroguanidin

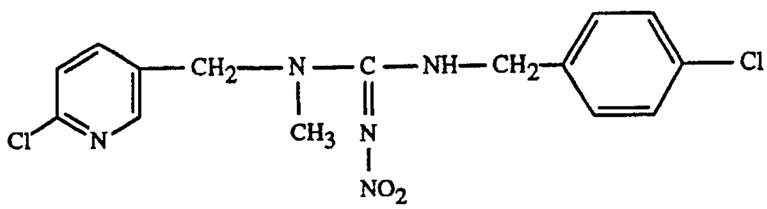

0,7 g 55 %iges Natriumhydrid wird bei 10-15°C in kleinen Portionen zu einer Lösung von 4,0g 1-(2-Chlorpyrid-5-ylmethyl)-1-methyl-2-nitroguanidin in 50 ml Dimethylformamid zugesetzt. Nach 20 Minuten lässt man bei 10- 15°C eine Lösung von 2,65 g 4-Chlorbenzylchlorid in 5 ml Dimethylformamid zutropfen. Das Reaktionsgemisch wird für 20 Stunden bei Raumtemperatur gerührt und anschliessend auf 200 ml Eiswasser gegossen. Die wässrige Lösung wird mit Chloroform extrahiert. Die organische Schicht wird abgetrennt, getrockenet und eingedampft. Nach säulenchromatographischer Reinigung an Kieselgel erhält man 1-(2-Chlorpyrid-5-ylmethyl)-2-(4-chlorbenzyl)-1-methyl-3-nitroguanidin mit einem Schmelzpunkt von 130-132°C.

In analoger Weise können die in der folgenden Tabelle aufgelisteten Verbindungen der Formel I hergestellt werden.

Tabelle 1:

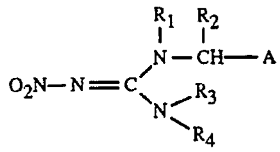

| Verb. Nr. | A | $R_1$ | $R_2$ | $R_3$ | $R_4$ | phys. Daten |
|---|---|---|---|---|---|---|
| 1.01 | 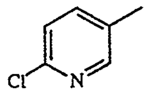 | $CH_3$ | H | H | H | Smp.163-165°C |
| 1.02 | 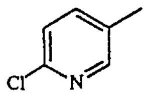 | H | H | H | H | Smp.195-197°C |
| 1.03 | 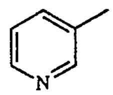 | H | H | $CH_3$ | H | Smp.162-163°C |
| 1.04 | 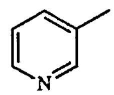 | $CH_3$ | H | H | H | Smp.134-136°C |
| 1.05 | 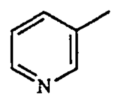 | $CH_3$ | H | $CH_3$ | H | Harz, $^1$H-NMR ($CDCl_3$): 2.96 (s,3H), 3.04 (s,3H), 4.70 (s,2H), 8.10 (breites s,1H) |
| 1.06 | 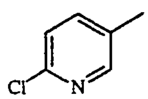 | $CH_3$ | H | $CH_3$ | H | Smp.129-131°C |
| 1.07 | 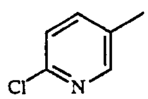 | H | H | $C_2H_5$ | H | Smp.125-127°C |
| 1.08 | 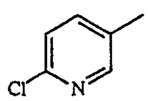 | H | H | $C_3H_7$-n | H | |

| Verb. Nr. | A | $R_1$ | $R_2$ | $R_3$ | $R_4$ | phys. Daten |
|---|---|---|---|---|---|---|
| 1.09 | 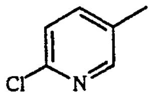 | H | H | 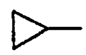 | H | |
| 1.10 | 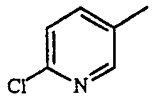 | H | H | $C_3H_7$-i | H | |
| 1.11 | 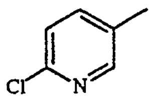 | H | H | $C_4H_9$-n | H | Smp.88-90°C |
| 1.12 | 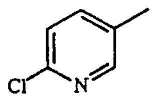 | H | H | $C_4H_9$-s | H | |
| 1.14 | 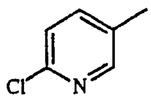 | H | H | $CH_3$ | $CH_3$ | Smp.158-160°C |
| 1.15 | 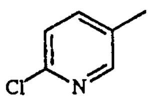 | H | H | $CH_3$ | $C_2H_5$ | Smp.115-119°C |
| 1.16 | 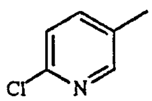 | H | H | $CH_3$ | $C_4H_9$-n | |
| 1.17 | 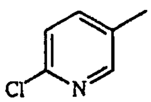 | H | H | $C_2H_5$ | $C_2H_5$ | Harz |
| 1.18 | 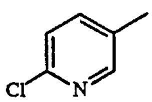 | H | H | $C_4H_9$-n | $C_4H_9$-n | |
| 1.19 | 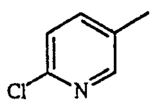 | H | H | $-CH_2CH_2-CH_2CH_2-$ | | |
| 1.20 | 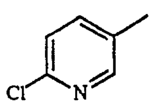 | H | H | H | 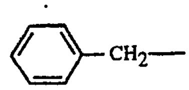 | Smp.154-155°C |
| 1.21 | 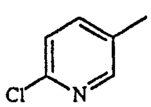 | H | H | H | 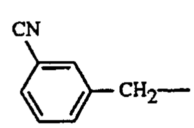 | |

| Verb. Nr. | A | $R_1$ | $R_2$ | $R_3$ | $R_4$ | phys. Daten |
|---|---|---|---|---|---|---|
| 1.22 | 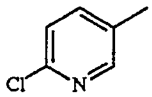 | H | H | H | 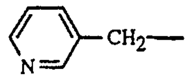 | |
| 1.23 | 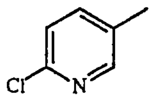 | H | H | H | 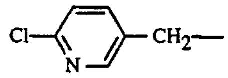 | |
| 1.24 | 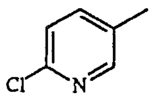 | $C_2H_5$ | H | H | H | Smp.136-138°C |
| 1.25 | 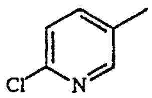 | 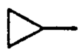 | H | H | H | |
| 1.26 | 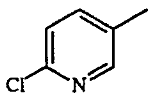 | $C_3H_7$-n | H | H | H | |
| 1.27 | 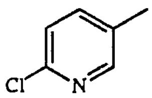 | $C_2H_5$ | H | $CH_3$ | H | Smp.107-109°C |
| 1.28 | 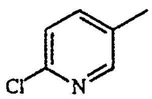 | 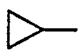 | H | $CH_3$ | H | |
| 1.29 | 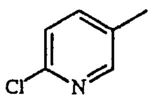 | $CH_3$ | H | $C_2H_5$ | H | |
| 1.30 | 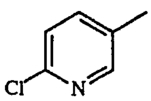 | $C_2H_5$ | H | $C_2H_5$ | H | |
| 1.31 | 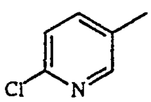 | 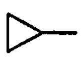 | H | $C_2H_5$ | H | |
| 1.32 | 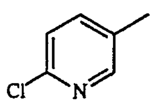 | $CH_3$ | H | $C_3H_7$-n | H | |
| 1.33 | 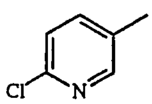 | $C_2H_5$ | H | $C_3H_7$-n | H | |

| Verb. Nr. | A | $R_1$ | $R_2$ | $R_3$ | $R_4$ | phys. Daten |
|---|---|---|---|---|---|---|
| 1.34 | 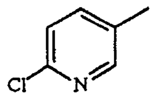 | $CH_3$ | H | 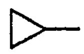 | H | |
| 1.35 | 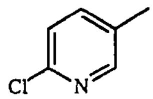 | $C_2H_5$ | H | 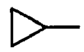 | H | |
| 1.36 | 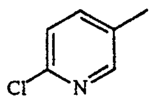 | $CH_3$ | H | $C_3H_7$-i | H | |
| 1.37 | 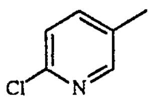 | 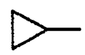 | H | $C_3H_7$-i | H | |
| 1.38 | 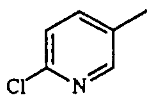 | $CH_3$ | H | $CH_3$ | $CH_3$ | |
| 1.39 | 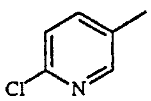 | $C_2H_5$ | H | $CH_3$ | $CH_3$ | |
| 1.40 | 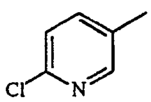 | $CH_3$ | H | $C_2H_5$ | $C_2H_5$ | |
| 1.41 | 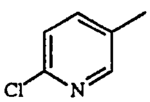 | H | H | $CH_3$ | 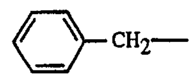 | |
| 1.42 | 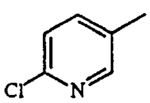 | $CH_3$ | H | H | 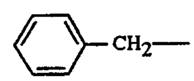 | |
| 1.43 | 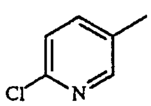 | $CH_3$ | H | H | 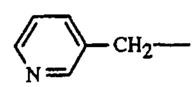 | |
| 1.44 | 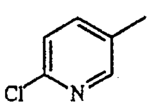 | $CH_3$ | H | H | 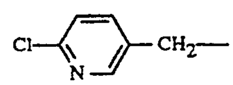 | |
| 1.45 | 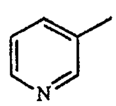 | $C_2H_5$ | H | H | H | |

| Verb. Nr. | A | $R_1$ | $R_2$ | $R_3$ | $R_4$ | phys. Daten |
|---|---|---|---|---|---|---|
| 1.55 | 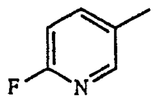 | $CH_3$ | H | H | H | |
| 1.56 | 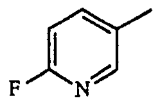 | $CH_3$ | H | $CH_3$ | H | |
| 1.57 | 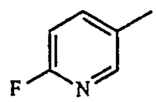 | H | H | $CH_3$ | $CH_3$ | |
| 1.58 | 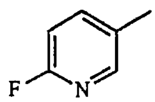 | 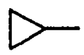 | H | H | H | |
| 1.59 | 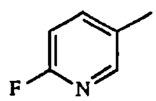 | $C_2H_5$ | H | H | H | |
| 1.60 | 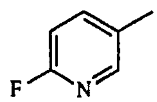 | $C_2H_5$ | H | $CH_3$ | H | |
| 1.61 | 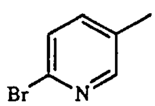 | $CH_3$ | H | H | H | |
| 1.62 | 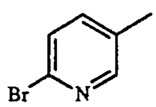 | $CH_3$ | H | $CH_3$ | H | |
| 1.63 | 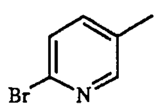 | $C_2H_5$ | H | H | H | |
| 1.64 | 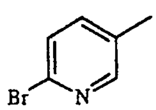 | $C_2H_5$ | H | $CH_3$ | H | |
| 1.65 | 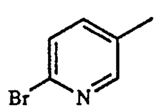 | H | H | $CH_3$ | $CH_3$ | |
| 1.66 | 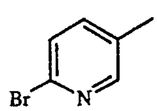 | 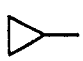 | H | $CH_3$ | H | |

| Verb. Nr. | A | $R_1$ | $R_2$ | $R_3$ | $R_4$ | phys. Daten |
|---|---|---|---|---|---|---|
| 1.67 | 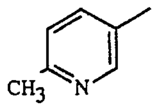 | $CH_3$ | H | H | H | |
| 1.68 | 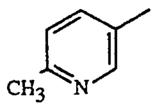 | $CH_3$ | H | $CH_3$ | H | |
| 1.69 | 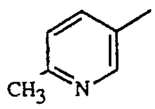 | $C_2H_5$ | H | H | H | |
| 1.70 | 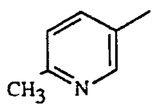 | $C_2H_5$ | H | $CH_3$ | H | |
| 1.71 | 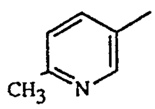 | H | H | $CH_3$ | $CH_3$ | |
| 1.72 | 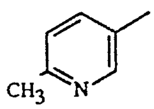 | 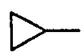 | H | $CH_3$ | H | |
| 1.73 | 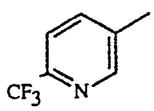 | $CH_3$ | H | H | H | |
| 1.74 | 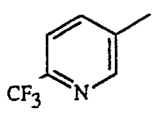 | $CH_3$ | H | $CH_3$ | H | |
| 1.75 | 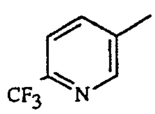 | $C_2H_5$ | H | H | H | |
| 1.76 | 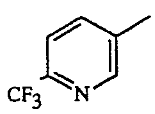 | $C_2H_5$ | H | $CH_3$ | H | |

| Verb. Nr. | A | $R_1$ | $R_2$ | $R_3$ | $R_4$ | phys. Daten |
|---|---|---|---|---|---|---|
| 1.77 | 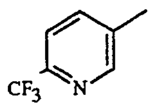 | H | H | $CH_3$ | $CH_3$ | |
| 1.78 | 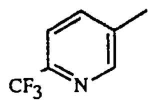 | 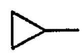 | H | H | H | |
| 1.79 | 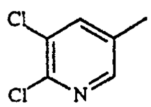 | $CH_3$ | H | H | H | |
| 1.80 | 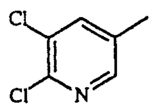 | $CH_3$ | H | $CH_3$ | H | |
| 1.81 | 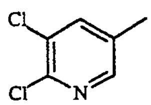 | $C_2H_5$ | H | H | H | |
| 1.82 | 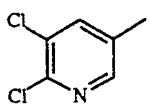 | $C_2H_5$ | H | $CH_3$ | H | |
| 1.83 | 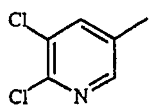 | H | H | $CH_3$ | $CH_3$ | |
| 1.84 | 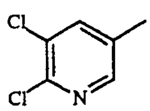 | 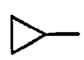 | H | H | H | |
| 1.85 | 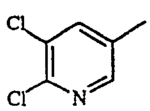 | 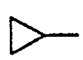 | H | $CH_3$ | H | |
| 1.86 | 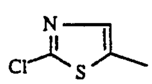 | $CH_3$ | H | H | H | Smp.112-114°C |
| 1.87 | 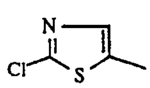 | $CH_3$ | H | $CH_3$ | H | |

| Verb. Nr. | A | $R_1$ | $R_2$ | $R_3$ | $R_4$ | phys. Daten |
|---|---|---|---|---|---|---|
| 1.88 | 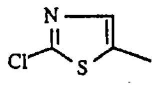 | $C_2H_5$ | H | H | H | |
| 1.89 | 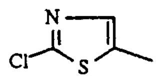 | $C_2H_5$ | H | $CH_3$ | H | |
| 1.90 | 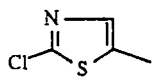 | $C_2H_5$ | H | $C_2H_5$ | H | |
| 1.91 | 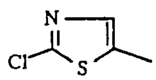 | H | H | $CH_3$ | $CH_3$ | Smp.159-160°C |
| 1.92 | 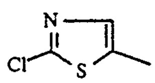 | H | H | $CH_3$ | 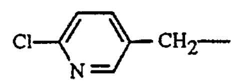 | |
| 1.93 | 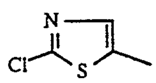 | H | H | $CH_3$ | H | Smp.168-170°C |
| 1.94 | 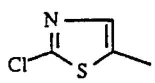 | 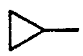 | H | H | H | |
| 1.95 | 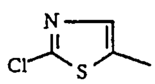 | 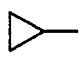 | H | $CH_3$ | H | |
| 1.96 | 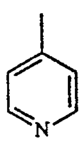 | $CH_3$ | H | H | H | |
| 1.97 | 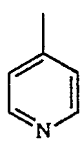 | $CH_3$ | H | $CH_3$ | H | |
| 1.98 | 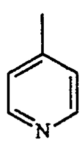 | 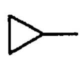 | H | H | H | |

| Verb. Nr. | A | $R_1$ | $R_2$ | $R_3$ | $R_4$ | phys. Daten |
|---|---|---|---|---|---|---|
| 1.99 | 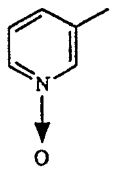 | $CH_3$ | H | H | H | Smp.203-205°C |
| 1.100 | 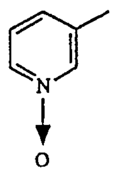 | H | H | $CH_3$ | $CH_3$ | |
| 1.101 | 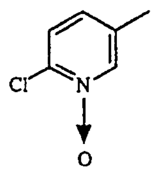 | H | H | $CH_3$ | $CH_3$ | |
| 1.102 | 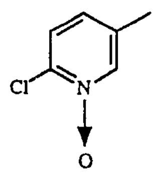 | $CH_3$ | H | $CH_3$ | H | |
| 1.103 | 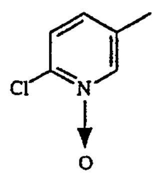 | $C_2H_5$ | H | $CH_3$ | H | |
| 1.104 | 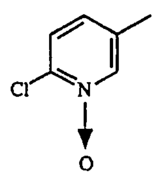 | 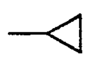 | H | $CH_3$ | H | |
| 1.105 | 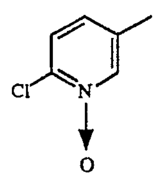 | $CH_3$ | H | H | H | Smp.198-200°C |

| Verb. Nr. | A | $R_1$ | $R_2$ | $R_3$ | $R_4$ | phys. Daten |
|---|---|---|---|---|---|---|
| 1.106 | 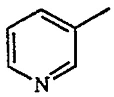 | H | H | H | H | Smp.201-202°C |
| 1.107 | 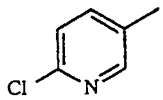 | H | H | $CH_3$ | H | Smp.148-150°C |
| 1.108 | 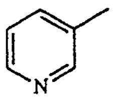 | H | H | $CH_3$ | $CH_3$ | Smp.145-146°C |
| 1.109 | 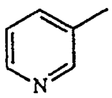 | H | H | $C_2H_5$ | H | Smp.113-116°C |
| 1.110 | 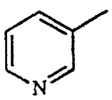 | H | $CH_3$ | H | H | Smp.158-160°C |
| 1.111 | 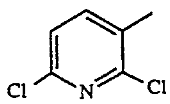 | $CH_3$ | H | H | H | Smp.210-212°C |
| 1.112 | 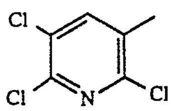 | $CH_3$ | H | H | H | Smp.202-203°C |
| 1.113 | 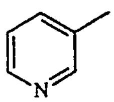 | H | $CH_3$ | $CH_3$ | H | Smp.161-163°C |
| 1.114 | 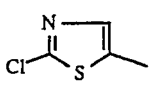 | H | H | H | H | Smp.158-160°C |
| 1.115 | 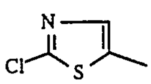 | H | H | $C_2H_5$ | H | Smp.135-136°C |
| 1.116 | 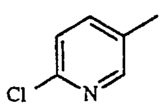 | 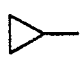 | $CH_3$ | H | H | |

| Verb. Nr. | A | $R_1$ | $R_2$ | $R_3$ | $R_4$ | phys. Daten |
|---|---|---|---|---|---|---|
| 1.117 | 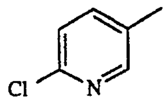 | $CH_3$ | $CH_3$ | $CH_3$ | H | |
| 1.118 | 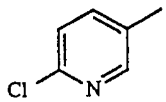 | $CH_3$ | $CH_3$ | $C_2H_5$ | H | |
| 1.119 | 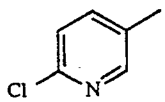 | $CH_3$ | $CH_3$ | 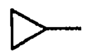 | H | |
| 1.120 | 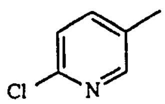 | $CH_3$ | $CH_3$ | $C_3H_7$-n | H | |
| 1.121 | 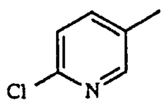 | H | $CH_3$ | $CH_3$ | H | |
| 1.122 | 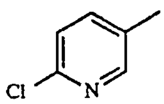 | H | $CH_3$ | $C_2H_5$ | H | |
| 1.123 | 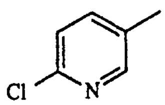 | H | $CH_3$ | 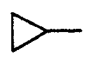 | H | |
| 1.124 | 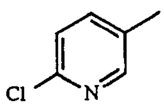 | H | $CH_3$ | $C_3H_7$-n | H | |
| 1.125 | 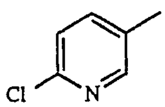 | H | $C_2H_5$ | $CH_3$ | $CH_3$ | |
| 1.126 | 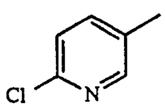 | H | $CH_3$ | $CH_3$ | $CH_3$ | |
| 1.127 | 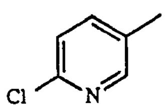 | H | $C_2H_5$ | $CH_3$ | H | |
| 1.128 | 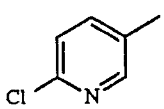 | H | $C_2H_5$ | $C_2H_5$ | H | |

| Verb. Nr. | A | $R_1$ | $R_2$ | $R_3$ | $R_4$ | phys. Daten |
|---|---|---|---|---|---|---|
| 1.129 | 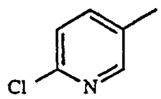 | $CH_3$ | $C_2H_5$ | H | H | |
| 1.130 | 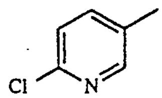 | $CH_2$ | $C_2H_5$ | $CH_3$ | H | |
| 1.131 | 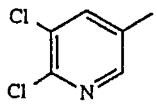 | H | H | $CH_3$ | H | Smp.173-175°C |
| 1.132 | 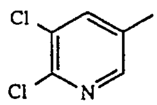 | H | H | $C_2H_5$ | H | Smp.159-161°C |
| 1.133 | 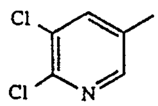 | H | H | 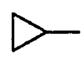 | H | |
| 1.134 | 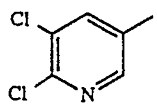 | H | H | $C_3H_7$-n | H | |
| 1.135 | 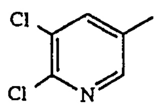 | H | H | $CH_3$ | $C_2H_5$ | |
| 1.136 | 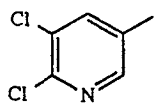 | $CH_3$ | H | $C_2H_5$ | H | |
| 1.137 | 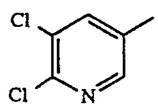 | $CH_3$ | H | $C_3H_7$-n | H | |
| 1.138 | 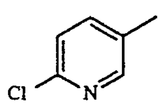 | H | H | $-(CH_2)_4-$ | | |
| 1.139 | 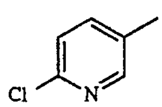 | H | H | $-(CH_2)_5-$ | | |
| 1.140 | 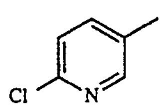 | H | $CH_3$ | $-CH_2)_4-$ | | |

| Verb. Nr. | A | $R_1$ | $R_2$ | $R_3$ | $R_4$ | phys. Daten |
|---|---|---|---|---|---|---|
| 1.141 | 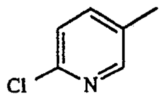 | H | $CH_3$ | $-(CH_2)_5-$ | | |
| 1.142 | 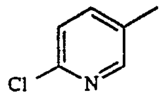 | H | H | 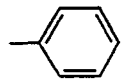 | H | |
| 1.143 | 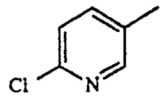 | $CH_3$ | H | 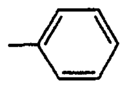 | H | |
| 1.144 | 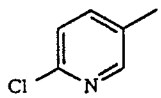 | H | H | 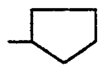 | H | |
| 1.145 | 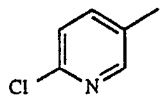 | $CH_3$ | H | 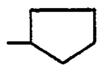 | H | |
| 1.146 | 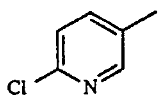 | $C_2H_5$ | H | 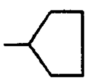 | H | |
| 1.147 | 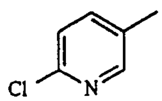 | $C_2H_5$ | H | 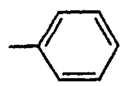 | H | |
| 1.148 | 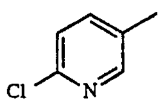 | H | $CH_3$ | H | $-CH_2-$ 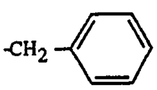 | |
| 1.149 | 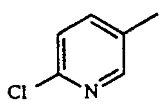 | H | $CH_3$ | H | $-CH_2-$ 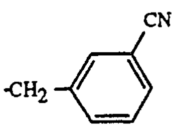 | |
| 1.150 | 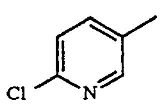 | H | $CH_3$ | H | $-CH_2-$ 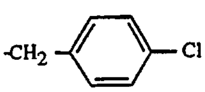 | |
| 1.151 | 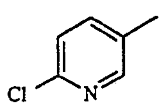 | $C_3H_7$-n | H | H | H | |

| Verb. Nr. | A | $R_1$ | $R_2$ | $R_3$ | $R_4$ | phys. Daten |
|---|---|---|---|---|---|---|
| 1.152 | 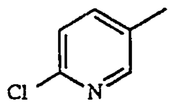 | $C_3H_7$-n | H | $CH_3$ | H | |
| 1.153 | 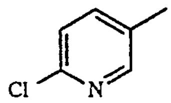 | $C_3H_7$-n | H | $C_2H_5$ | H | |
| 1.154 | 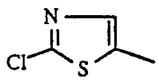 | $C_3H_7$-n | H | H | H | |
| 1.155 | 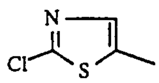 | $C_3H_7$-n | H | $CH_3$ | H | |
| 1.156 | 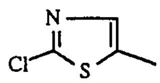 | $C_3H_7$-n | H | $C_2H_5$ | H | |
| 1.157 | 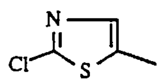 | H | H | H | $-CH_2$ 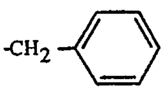 | |
| 1.158 | 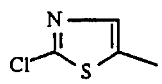 | H | H | H | $-CH_2$ 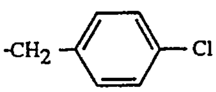 | |
| 1.159 | 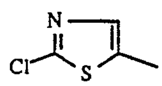 | $CH_3$ | H | H | $-CH_2$ 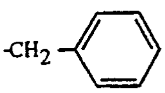 | |
| 1.160 | 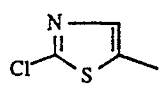 | $CH_3$ | H | H | $-CH_2$ 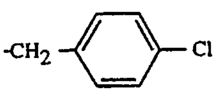 | |
| 1.161 | 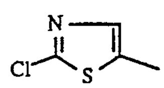 | $C_3H_7$-i | H | H | H | |
| 1.162 | 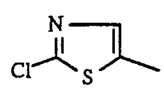 | $C_3H_7$-i | H | $CH_3$ | H | |
| 1.163 | 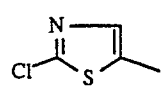 | $C_3H_7$-i | H | $C_2H_5$ | H | |

| Verb. Nr. | A | $R_1$ | $R_2$ | $R_3$ | $R_4$ | phys. Daten |
|---|---|---|---|---|---|---|
| 1.164 | 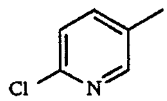 | $C_3H_7$-i | H | $CH_3$ | H | |
| 1.165 | 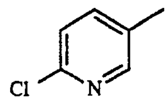 | $C_3H_7$-i | H | $C_2H_5$ | H | |
| 1.166 | 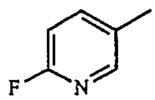 | $CH_3$ | H | H | H | |
| 1.167 | 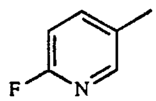 | $C_2H_5$ | H | H | H | |
| 1.168 | 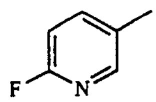 | H | H | $CH_3$ | H | |
| 1.169 | 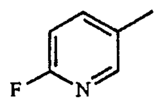 | H | H | $C_2H_5$ | H | |
| 1.170 | 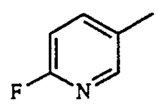 | H | H | $CH_3$ | $CH_3$ | |
| 1.171 | 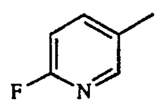 | H | H | $CH_3$ | $C_2H_5$ | |
| 1.172 | 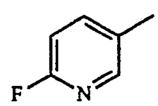 | $CH_3$ | H | $CH_3$ | H | |
| 1.173 | 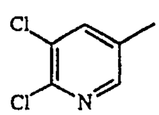 | H | H | H | H | Smp.207-209°C |
| 1.174 | 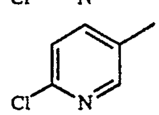 | $CH_3$ | H | H | 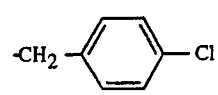 | Smp.130-132°C |
| 1.175 | 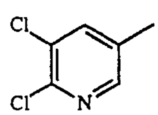 | H | H | $C_4H_9$-n | H | Smp.152-153°C |

| Verb. Nr. | A | $R_1$ | $R_2$ | $R_3$ | $R_4$ | phys. Daten |
|---|---|---|---|---|---|---|
| 1.176 | 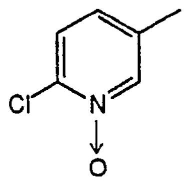 | H | H | $CH_3$ | H | |

Formulierungsbeispiele (% = Gewichtsprozent)

| Beispiel F1: Emulsions-Konzentrate | a) | b) | c) |
|---|---|---|---|
| Wirkstoff Nr. 1.05 | 25 % | 40 % | 50 % |
| Ca-Dodecylbenzolsulfonat | 5 % | 8 % | 6 % |
| Ricinusölpolyäthylenglykoläther (36 Mol AeO) | 5 % | - | - |
| Tributylphenolpolyäthylenglykoläther (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| Beispiel F2: Lösungen | a) | b) | c) | d) |
|---|---|---|---|---|
| Wirkstoff Nr. 1.05 | 80 % | 10 % | 5 % | 95 % |
| Aethylenglykolmonomethyläther | 20 % | - | - | - |
| Polyäthylenglykol MG 400 | - | 70 % | - | - |
| N-Methyl-2-pyrrolidon | - | 20 % | - | - |
| Epoxidiertes Kokosnussöl | - | - | 1 % | 5 % |
| Benzin (Siedegrenzen 160-190 °C) | - | - | 94 % | - |

Die Lösungen sind zur Anwendung in Form kleinster Tropfen geeignet.

| Beispiel F3: Granulate | a) | b) | c) | d) |
|---|---|---|---|---|
| Wirkstoff Nr. 1.05 | 5 % | 10 % | 8 % | 21 % |
| Kaolin | 94 % | - | 79 % | 54 % |
| Hochdisperse Kieselsäure | 1 % | - | 13 % | 7 % |
| Attapulgit | - | 90 % | - | 18 % |

Der Wirkstoff wird in Methylenchlorid gelöst, auf den Träger aufgesprüht und das Lösungsmittel anschliessend im Vakuum abgedampft.

| Beispiel F4: Stäubemittel | a) | b) |
|---|---|---|
| Wirkstoff Nr. 1.05 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

| Beispiel F5: Spritzpulver | a) | b) | c) |
|---|---|---|---|
| Wirkstoff Nr. 1.01 oder 1.03 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 62 % | 27 % | - |

Der Wirkstoff oder die Wirkstoffkombination wird mit den Zusatzstoffen vermischt und in einer geeigneten Mühle gut vermahlen. Man erhält Spritzpulver, die sich mit Wasser zu Suspensionen jeder gewünschten Konzentration verdünnen lassen.

| Beispiel F6: Emulsions-Konzentrat | |
|---|---|
| Wirkstoff Nr. 1.01 oder 1.03 | 10 % |
| Octylphenolpolyäthylenglykoläther (4-5 Mol AeO) | 3 % |
| Ca-Dodecylbenzolsulfonat | 3 % |
| Ricinusölpolyglykoläther (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| Beispiel F7: Stäubemittel | a) | b) |
|---|---|---|
| Wirkstoff Nr. 1.02 | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

| Beispiel F8: Extruder-Granulat | |
|---|---|
| Wirkstoff Nr. 1.06 | 10 % |
| Na-Ligninsulfonat | 2 % |
| Carboxymethylcellulose | 1 % |
| Kaolin | 87 % |

Der Wirkstoff oder die Wirkstoffkombination wird mit den Zusatzstoffen vermischt, vermahlen und mit Wasser angefeuchtet. Dieses Gemisch wird extrudiert, granuliert und anschliessend im Luftstrom getrocknet.

| Beispiel F9: Umhüllungs-Granulat | |
|---|---|
| Wirkstoff Nr. 1.04 | 3 % |
| Polyäthylenglykol (MG 200) | 3 % |
| Kaolin | 94 % |

Der fein gemahlene Wirkstoff oder die Wirkstoffkombination wird in einem Mischer auf das mit Polyäthylenglykol angefeuchtete Kaolin gleichmässig aufgetragen. Auf diese Weise erhält man staubfreie Umhüllungs-Granulate.

| Beispiel F10: Suspensions-Konzentrat | |
|---|---|
| Wirkstoff Nr. 1.01 | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| Silikonöl in Form einer 75 %igen wässrigen Emulsion | 1 % |
| Wasser | 32 % |

Der fein gemahlene Wirkstoff oder die Wirkstoffkombination wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

## Biologische Beispiele

### Beispiel B1: Wirkung gegen Nilaparvata lugens

Reispflanzen werden mit einer wässrigen Emulsions-Spritzbrühe, die 400 ppm des Wirkstoffes enthält, behandelt. Nach dem Antrocknen des Spritzbelages werden die Reispflanzen mit Zikadenlarven des 2. und 3. Stadiums besiedelt. 21 Tage später erfolgt die Auswertung. Aus dem Vergleich der Anzahl überlebender Zikaden auf den behandelten zu denjenigen auf den unbehandelten Pflanzen wird die prozentuale Reduktion der Population (% Wirkung) bestimmt.

Die Verbindungen der Tabelle 1 zeigen eine gute Wirkung gegen Nilaparvata lugens in diesem Test. Insbesondere die Verbindungen 1.01, 1.03, 1.04, 1.05, 1.07, 1.14, 1.15, 1.24, 1.86, 1.93, 1.107, 1.108, 1.113 und 1.115 zeigen eine Wirkung über 80%.

### Beispiel B2: Wirkung fegen Nephotettix cincticeps

Reispflanzen werden mit einer wässrigen Emulsions-Spritzbrühe, die 400 ppm des Wirkstoffes enthält, behandelt. Nach dem Antrocknen des Spritzbelages werden die Reispflanzen mit Zikadenlarven des 2. und 3. Stadiums besiedelt. 21 Tage später erfolgt die Auswertung. Aus dem Vergleich der Anzahl überlebender Zikaden auf den behandelten zu denjenigen auf den unbehandelten Pflanzen wird die prozentuale Reduktion der Population (% Wirkung) bestimmt.

Die Verbindungen der Tabelle 1 zeigen eine gute Wirkung gegen Nephotettix cincticeps in diesem Test. Insbesondere die Verbindung 1.01, 1.06, 1.14, 1.15 und 1.91 zeigen eine Wirkung über 80 %.

Beispiel B3: Wirkung gegen Diabrotica balteata Larven

Maiskeimlinge werden mit einer wässrigen Emulsions-Spritzbrühe, die 400 ppm des Wirkstoffes enthält, besprüht. Nach dem Antrocknen des Spritzbelages werden die Maiskeimlinge mit 10 Larven des zweiten Stadiums von Diabrotica balteata besiedelt und in einen Plastikbehälter gegeben. 6 Tage später erfolgt die Auswertung. Aus dem Vergleich der Anzahl toter Larven auf den behandelten zu denjenigen auf den unbehandelten Pflanzen wird die prozentuale Reduktion der Population (% Wirkung) bestimmt.

Die Verbindungen der Tabelle 1 zeigen eine gute Wirkung gegen Diabrotica balteata in diesem Test. Insbesondere die Verbindung 1.01 zeigt eine Wirkung über 80 %.

Beispiel B4: Wirkung geben Heliothis virescens Raupen

Junge Sojapflanzen werden mit einer wässrigen Emulsions-Spritzbrühe, die 400 ppm des Wirkstoffes enthält, besprüht. Nach dem Antrocknen des Spritzbelages werden die Sojapflanzen mit 10 Raupen des ersten Stadiums von Heliothis virescens besiedelt und in einen Plastikbehälter gegeben. 6 Tage später erfolgt die Auswertung. Aus dem Vergleich der Anzahl toter Raupen und des Frasschadens auf den behandelten zu denjenigen auf den unbehandelten Pflanzen wird die prozentuale Reduktion der Population bezw. die prozentuale Reduktion des Frasschadens (% Wirkung) bestimmt.

Die Verbindungen der Tabelle 1 zeigen eine gute Wirkung gegen Heliothis virescens in diesem Test. Insbesondere die Verbindung 1.01, 1.107. und 1.110 zeigen eine Wirkung über 80 %.

Beispiel B5: Wirkung fegen Spodoptera littoralis Raupen

Junge Sojapflanzen werden mit einer wässrigen Emulsions-Spritzbrühe, die 400 ppm des Wirkstoffes enthält, besprüht. Nach dem Antrocknen des Spritzbelages werden die Sojapflanzen mit 10 Raupen des dritten Stadiums von Spodoptera littoralis besiedelt und in einen Plastikbehälter gegeben. 3 Tage später erfolgt die Auswertung. Aus dem Vergleich der Anzahl toter Raupen und des Frasschadens auf den behandelten zu denjenigen auf den unbehandelten Pflanzen wird die prozentuale Reduktion der Population bezw. die prozentuale Reduktion des Frasschadens (% Wirkung) bestimmt.

Die Verbindungen der Tabelle 1 zeigen eine gute Wirkung gegen Spodoptera littoralis in diesem Test. Insbesondere die Verbindungen 1.01, 1.03, 1.07, 1.14, 1.86, 1.107 und 1.108 zeigen eine Wirkung über 80 %.

Beispiel B6: Wirkung fegen Aphis craccivora

Erbsenkeimlinge werden mit Aphis craccivora infiziert und anschliessend mit einer Spritzbrühe, die 400 ppm des Wirkstoffes enthält, besprüht und bei 20°C inkubiert. 3 und 6 Tage später erfolgt die Auswertung. Aus dem Vergleich der Anzahl toter Blattläuse auf den behandelten zu denjenigen auf den unbehandelten Pflanzen wird die prozentuale Reduktion der Population (% Wirkung) bestimmt.

Die Verbindungen der Tabelle 1 zeigen eine gute Wirkung gegen Aphis craccivora in diesem Test. Insbesondere die Verbindungen 1.01, 1.03, 1.04, 1.05, 1.06, 1.07, 1.14, 1.15, 1.24, 1.86, 1.91, 1.93, 1.99, 1.107, 1.114 und 1.115 zeigen eine Wirkung über 80 %.

Beispiel B7: Systemische Wirkung gegen Nilaparvata lugens

Töpfe mit Reispflanzen werden in eine wässrige Emulsions-Lösung, die 400 ppm des Wirkstoffes enthält, gestellt. Anschliessend werden die Reispflanzen mit Larven des 2. und 3. Stadiums besiedelt. 6 Tage später erfolgt die Auswertung. Aus dem Vergleich der Anzahl Zikaden auf den behandelten zu denjenigen auf den unbehandelten Pflanzen wird die prozentuale Reduktion der Population (% Wirkung) bestimmt.

Die Verbindungen der Tabelle 1 zeigen eine gute Wirkung gegen Nilaparvata lugens in diesem Test. Insbesondere die Verbindungen 1.01, 1.03, 1.04, 1.06, 1.07, 1.14, 1.15, 1.86, 1.93, 1.107, 1.114 und 1.115 zeigen eine Wirkung über 80 %.

Beispiel B8: Systemische Wirkung gegen Nephotettix cincticeps

Töpfe mit Reispflanzen werden in eine wässrige Emulsions-Lösung, die 400 ppm des Wirkstoffes enthält, gestellt. Anschliessend werden die Reispflanzen mit Larven des 2. und 3. Stadiums besiedelt. 6 Tage später erfolgt die Auswertung. Aus dem Vergleich der Anzahl Zikaden auf den behandelten zu denjenigen auf den unbehandelten Pflanzen wird die prozentuale Reduktion der Population (% Wirkung) bestimmt.

Die Verbindungen der Tabelle 1 zeigen eine gute Wirkung gegen Nephotettix cincticeps in diesem Test. Insbesondere die Verbindungen 1.01, 1.03, 1.04, 1.06, 1.14, 1.15, 1.86, 1.91 und 1.114 zeigen eine Wirkung über 80 %.

Beispiel B9: Systemische Wirkung gegen Myzus persicae

Erbsenkeimlinge werden mit Myzus persicae infiziert, anschliessend mit den Wurzeln in eine Spritzbrühe, die 400 ppm des Wirkstoffes enthält, gestellt und bei 20 °C inkubiert. 3 und 6 Tage später erfolgt die Auswertung. Aus dem Vergleich der Anzahl toter Blattläuse auf den behandelten zu denjenigen auf den unbehandelten Pflanzen wird die prozentuale Reduktion der Population (% Wirkung) bestimmt.

Die Verbindungen der Tabelle 1 zeigen eine gute Wirkung gegen Myzus persicae in diesem Test. Insbesondere die Verbindung 1.01, 1.06, 1.14 und 1.86 zeigen eine Wirkung über 80 %.

Beispiel B10: Wirkung gegen Anthonomus grandis Adulte

Junge Baumwollpflanzen werden mit einer wässrigen Emulsions-Spritzbrühe, die 400 ppm des Wirkstoffes enthält, besprüht. Nach dem Antrocknen des Spritzbelages werden die Baumwollpflanzen mit 10 Adulten von Anthonomus grnadis besiedelt und in einen Plastikbehälter gegeben. 3 Tage später erfolgt die Auswertung. Aus dem Vergleich der Anzahl toter Käfer und des Frasschadens auf den behandelten zu denjenigen auf den unbehandelten Pflanzen wird die prozentuale Reduktion der Population bzw. die prozentuale Reduktion des Frasschadens (% Wirkung) bestimmt.

Die Verbindungen der Tabelle 1 zeigen eine gute Wirkung gegen Anthonomus grandis in diesem Test. Insbesondere die Verbindung 1.01, 1.14, 1.15, 1.86, 1.91 und 1.93 zeigen eine Wirkung über 80 %.

Beispiel B11: Wirkung gegen Bemisia tabaci

Buschbohnen-Pflanzen werden in Gazekäfige gestellt und mit Adulten Bemisia tabaci (Weisse Fliege) besiedelt. Nach erfolgter Eiablage werden alle Adulten entfernt und 10 Tage später die Pflanzen mit den darauf befindenden Nymphen mit einer wässrigen Emulsions-Spritzbrühe der zu prüfenden Wirkstoffe (Konzentration 400 ppm) behandelt. Die Auswertung erfolgt 14 Tage nach der Wirkstoff-Applikation auf %-Schlupf im Vergleich zu den unbehandelten Kontrollansätzen.

Verbindungen gemäss Tabelle 1 zeigen in diesem Test gute Wirkung gegen Bemisia tabaci. Insbesondere die Verbindungen 1.01, 1.06, 1.07, 1.14, 1.86, 1.107 und 1.108 zeigen eine Wirkung über 80 %.

Beispiel B12: Wirkung gegen Schmeissfliegen Lucilia cuprina

Frisch abgelegte Eier der Schmeissfliegenart Lucilia cuprina werden in kleinen Portionen (30-50 Eier) in

Reagenzgläser gegeben, in denen zuvor 4 ml Nährmedium mit 1 ml Testlösung, die 16 ppm des zu prüfenden Wirkstoffes enthält, vermischt worden sind. Nach Beimpfung des Kulturmediums werden die Testgläser mit einem Wattestopfen verschlossen und im Brutschrank bei 30 °C 4 Tage lang bebrütet. Im unbehandelten Medium entwickeln sich bis zu diesem Zeitpunkt ca. 1 cm lange Larven (Stadium 3). Ist die Substanz aktiv, so sind die Larven zu diesem Zeitpunkt entweder tod oder deutlich zurückgeblieben. Die Auswertung erfolgt nach 96 Stunden.

Verbindungen der Tabelle 1 zeigen eine gute Wirkung gegen Lucilia cuprina in diesem Test.

## Ansprüche

1. Nitroguanidine der Formel I

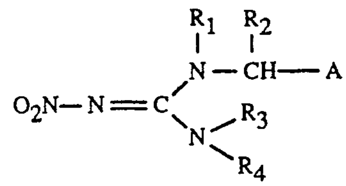

(I)

worin
$R_1$ für Wasserstoff, $C_1$-$C_4$-Alkyl oder $C_3$-$C_6$-Cycloalkyl,
$R_2$ für Wasserstoff oder $C_1$-$C_4$-Alkyl,
$R_3$ für Wasserstoff, $C_1$-$C_4$-Alkyl oder $C_3$-$C_6$-Cycloalkyl,
$R_4$ für Wasserstoff, $C_1$-$C_4$-Alkyl, $C_3$-$C_6$-Cycloalkyl oder ein Rest $-CHR_5$-B, oder
$R_3$ und $R_4$ gemeinsam für $-(CH_2)_4-$ oder $-(CH_2)_5-$,
$R_5$ für Wasserstoff oder $C_1$-$C_4$-Alkyl,
A für einen unsubstituierten oder ein- bis vierfach substituierten aromatischen oder nichtaromatischen, monocyclischen oder bicyclischen heterocyclischen Rest, wobei ein bis zwei Substituenten aus der Gruppe $C_1$-$C_3$-Halogenalkyl, Cyclopropyl, Halogencyclopropyl, $C_2$-$C_3$-Alkenyl, $C_2$-$C_3$-Alkinyl, $C_2$-$C_3$-Halogenalkenyl, $C_2$-$C_3$-Halogenalkinyl, $C_1$-$C_3$-Halogenalkoxy, $C_1$-$C_3$-Alkylthio, $C_1$-$C_3$-Halogenalkylthio, Allyloxy, Propargyloxy, Allylthio, Propargylthio, Halogenallyloxy, Halogenallylthio, Cyan und Nitro und ein bis vier Substituenten aus der Gruppe $C_1$-$C_3$-Alkyl, $C_1$-$C_3$-Alkoxy und Halogen ausgewahlt sind, und
B für Phenyl, Cyanophenyl, Nitrophenyl, Halogenphenyl mit 1 bis 3 Halogenatomen, 3-Pyridyl, 5-Thiazolyl, durch ein bis zwei Substituenten aus der Gruppe $C_1$-$C_3$-Alkyl, $C_1$-$C_3$-Halogenalkyl, Cyclopropyl, Halogencyclopropyl, $C_2$-$C_3$-Alkenyl, $C_2$-$C_3$-Alkinyl, $C_1$-$C_3$-Alkoxy, $C_2$-$C_3$-Halogenalkenyl, $C_2$-$C_3$-Halogenalkinyl, $C_1$-$C_3$-Halogenalkoxy, $C_1$-$C_3$-Alkylthio, $C_1$-$C_3$-Halogenalkylthio, Allyloxy, Propargyloxy, Allylthio, Propargylthio, Halogenallyloxy, Halogenallylthio, Halogen, Cyan und Nitro substituiertes 5-Thiazolyl; oder durch ein bis zwei Reste aus der Gruppe $C_1$-$C_3$-Halogenalkyl, Cyclopropyl, Halogencyclopropyl, $C_2$-$C_3$-Alkenyl, $C_2$-$C_3$-Alkinyl, $C_2$-$C_3$-Halogenalkenyl, $C_2$-$C_3$-Halogenalkinyl, $C_1$-$C_3$-Halogenalkoxy, $C_1$-$C_3$-Alkylthio, $C_1$-$C_3$-Halogenalkylthio, Allyloxy, Propargyloxy, Allylthio, Propargylthio, Halogenallyloxy, Halogenallylthio, Cyan und Nitro oder durch ein bis vier Reste aus der Gruppe $C_1$-$C_3$-Alkyl, $C_1$-$C_3$-Alkoxy oder Halogen substituiertes 3-Pyridyl stehen; sowie deren Salze mit anorganischen Säuren; unter Ausnahme der Verbindungen 1-Nitro-2-(pyrid3-ylmethyl)-guanidin, 1-Nitro-2-(pyrid-2-ylmethyl)-guanidin, 1-Nitro-2-(pyrid-4-ylmethyl)-guanidin, 1-Nitro-2-(1-oxo-pyrid-3-ylmethyl)-guanidin und 1-(Benzimidazol-2-ylmethyl)-2-nitroguanidin.

2. Verbindungen gemäss Anspruch 1, dadurch gekennzeichnet, dass der heterocyclische Rest A ungesättigt ist, über ein Kohlenstoffatom an den Grundkörper des Nitroguanidins der Formel I gebunden ist und mindestens ein Stickstoffatom enthält.

3. Verbindungen gemäss Anspruch 2, dadurch gekennzeichnet, dass der heterocyclische Rest A ungesättigt ist und über ein Kohlenstoffatom an den Grundkörper des Nitroguanidins gebunden ist und ein bis drei Heteroatome aus der Reihe Sauerstoff, Schwefel und Stickstoff enthält, wobei jeweils höchstens ein Sauerstoff- oder Schwefelatom enthalten ist.

4. Verbindungen gemäss Anspruch 3, dadurch gekennzeichnet, dass der heterocyclische Rest A ein bis drei Heteroatome aus der Reihe Sauerstoff, Schwefel und Stickstoff enthält, wovon stets ein Heteroatom Stickstoff ist und höchstens ein Sauerstoffatom oder Schwefelatom enthalten ist.

5. Verbindungen gemäss Anspruch 1, dadurch gekennzeichnet, dass der heterocyclische Rest A einen über

ein Kohlenstoffatom an den Guanidinkörper gebundenen Grundkörper aus der Gruppe

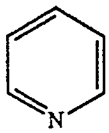 , 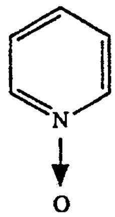 , 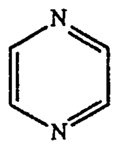 , 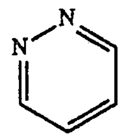 , 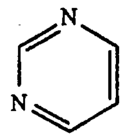 , 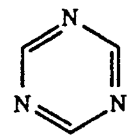 , 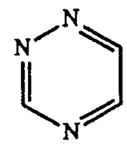 .

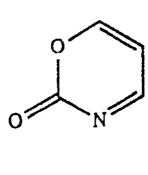 , 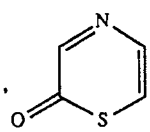 , 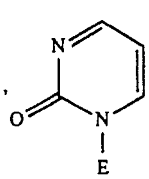 , 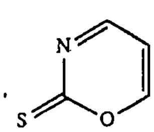 , 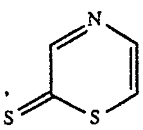 , 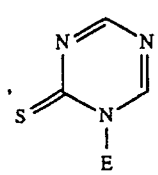 ,

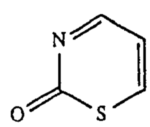 , 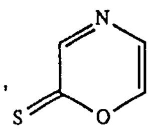 , 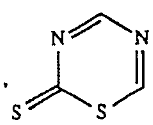 , 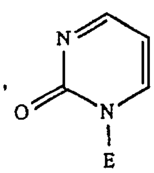 , 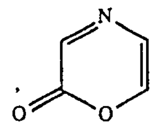 ,

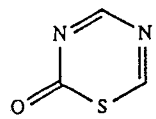 , 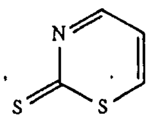 , 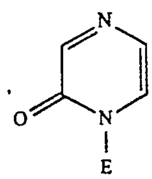 , 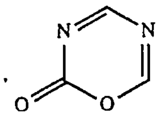 , 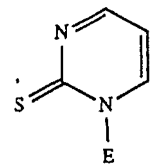 , 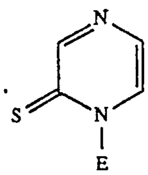 ,

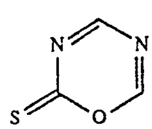 , 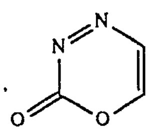 , 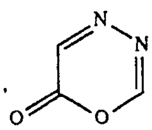 , 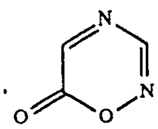 , 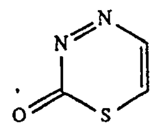 ,

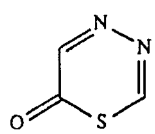 , 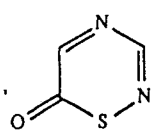 , 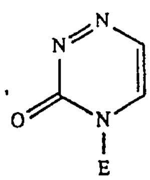 , 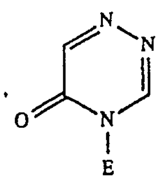 , 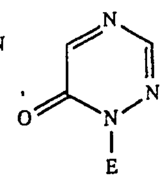 ,

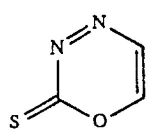 , 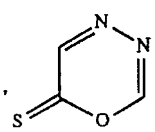 , 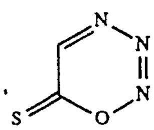 , 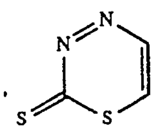 , 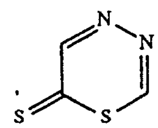 ,

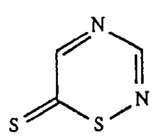 , 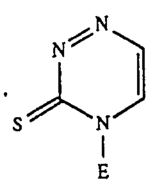 , 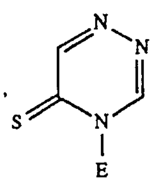 , 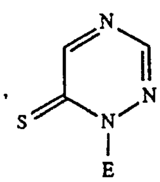 , 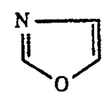 ,

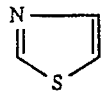 , 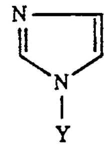 , 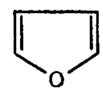 , 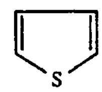 ,

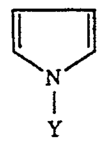 , 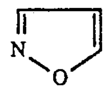 , 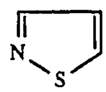 , 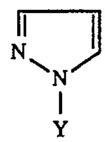 ,

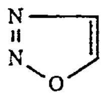 , 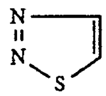 , 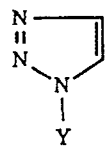 , 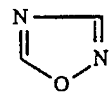 , 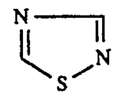 ,

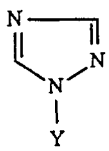 , 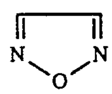 , 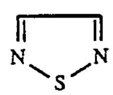 , 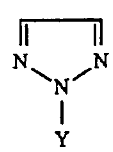 , 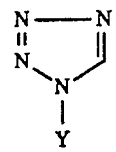 ,

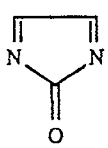 , 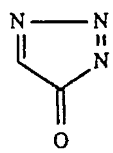 , 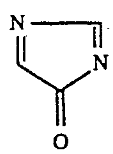 , 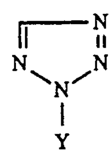 , 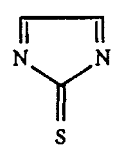 ,

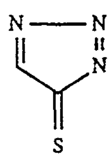 , 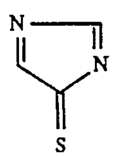 , 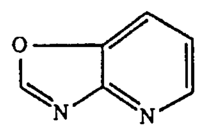 , 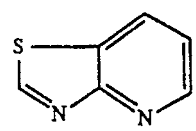 , 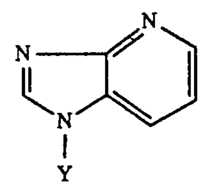 ,

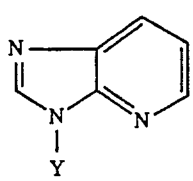 , 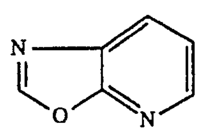 , 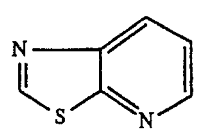 , 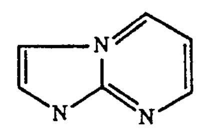 ,

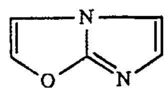 , 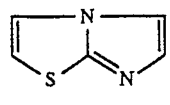 , 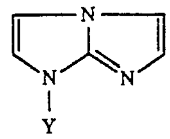 ,

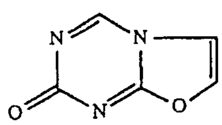 , 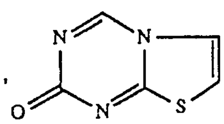 , 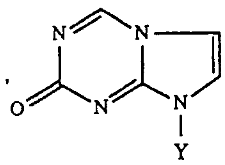 , 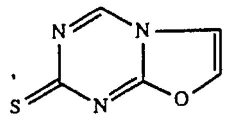 ,

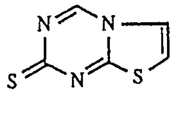 und 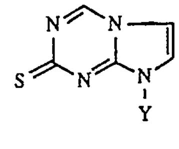

darstellt, welcher unsubstituiert ist oder je nach den Substitutionsmöglichkeiten des Ringsystems bis zu vier der im Anspruch 1 definierten Substituenten trägt und worin E für $C_1$-$C_3$-Alkyl und Y für Wasserstoff, $C_1$-$C_3$-Alkyl oder Cyclopropyl stehen.

6. Verbindungen gemäss Anspruch 5, dadurch gekennzeichnet, dass der heterocyclische Rest A unsubstituiert ist oder ein bis drei Substituenten aus der Gruppe Halogen, $C_1$-$C_3$-Alkyl, $C_1$-$C_3$-Halogenalkyl, $C_1$-$C_3$-Halogenalkoxy oder $C_1$-$C_3$-Alkoxy trägt.

7. Verbindungen gemäss Anspruch 6, dadurch gekennzeichnet, dass die heterocyclischen Reste A Pyridylreste oder Thiazolylreste sind.

8. Verbindungen gemäss Anspruch 1, dadurch gekennzeichnet, dass der Rest B für einen Phenyl-, Pyridyl- oder Thiazolylrest steht, welcher unsubstituiert oder durch ein bis zwei Reste aus der Gruppe Halogen, $C_1$-$C_3$-Alkyl, $C_1$-$C_3$-Halogenalkyl, $C_1$-$C_3$-Halogenalkoxy oder $C_1$-$C_3$-Alkoxy substituiert ist.

9. Verbindungen gemäss Anspruch 7, dadurch gekennzeichnet, dass A für 3-Pyridyl, 2-Halogenpyrid-5-yl, 2,3-Dihalogenpyrid-5-yl oder 2-Halogenthiazol-4-yl, 1-Oxopyrid-3-yl, 1-Oxo-2-halogenpyrid-5-yl oder 1-Oxo-2,3-dihalogenpyrid-5-yl steht.

10. Verbindungen gemäss Anspruch 1, dadurch gekennzeichnet, dass $R_1$ und $R_3$ unabhängig voneinander Wasserstoff, Methyl, Äthyl oder Cyclopropyl, $R_2$ Wasserstoff,$R_4$ Wasserstoff oder Methyl und A Pyridyl, 1-Oxopyridyl, Thiazolyl oder jeweils durch ein bis drei Substituenten aus der Gruppe Halogen, $C_1$-$C_3$-Alkyl, $C_1$-$C_3$-Halogenalkyl, $C_1$-$C_3$-Halogenalkoxy oder $C_1$-$C_3$-Alkoxy substituiertes Pyridyl, 1-Oxopyridyl oder Thiazolyl bedeuten.

11. Verbindungen gemäss Anspruch 1 ausgewählt aus der Gruppe 1-(2-Chlorpyrid-5-ylmethyl)-1-methyl-2-nitroguanidin, 1-(2-Chlorpyrid-5-ylmethyl)-2-nitroguanidin, 1-Methyl-2-nitro-3-(pyrid-3-ylmethyl)-guanidin, 1-Methyl-2-nitro-1-(pyrid-3-ylmethyl)-guanidin, 1,2-Dimethyl-3-nitro-1-(pyrid-3-ylmethyl)-guanidin, 1-(2-Chlorpyrid-5-ylmethyl)- 1,2-dimethyl-3-nitroguanidin, 1-(2-Chlorthiazol-5-ylmethyl)-1-methyl-2-nitroguanidin, 1-(2-Chlorpyrid-5-ylmethyl)-2,2-dimethyl-3-nitroguanidin, 1-(2-Chlorpyrid-5-ylmethyl)-2-methyl-3-nitroguanidin, 1,1-Dimethyl-2-nitro-3-(pyrid-3-ylmethyl)-guanidin, 1-Aethyl-2-nitro-3-(pyrid-3-methyl)-guanidin, 1-Aethyl-2(2-chlorpyrid-5-ylmethyl)-3-nitroguanidin, 1-Aethyl-1-methyl-2-(2-chlorpyrid-5-ylmethyl)-3-nitroguanidin, 1-(2-Chlorthiazol-5-ylmethyl)-2,2-dimethyl-3-nitroguanidin, 1-(2,3-Dichlorpyrid-5-ylmethyl)-2-methyl-3-nitroguanidin, 1-Aethyl-2-(2,3-dichlorpyrid-5-ylmethyl)-3-nitroguanidin, 1-(2,3-Dichlorpyrid-5-ylmethyl)-1-methyl-2-nitroguanidin, 1-Aethyl-1-(2,3-dichlorpyrid-5-ylmethyl)-2-nitroguanidin, 1-(2-Chlor-1-oxopyrid-5-ylmethyl)-1-methyl-2-nitroguanidin, 1-Aethyl-1-(2-chlor-1-oxopyrid-5-ylmethyl)-2-nitroguanidin, 1-(2-Chlor-1-oxopyrid-5-ylmethyl)-2-methyl-3-nitroguanidin, 1-Aethyl-1-(2-chlor-1-oxopyrid-5-ylmethyl)-3-nitroguanidin, 1-(2-Chlorthiazol-5-ylmethyl)-2-methyl-3-nitroguanidin, 1-(2-Chlorthiazol-5-ylmethyl)-2-nitroguanidin und 1-Aethyl-2-(2-chlorthiazol-5-ylmethyl)-3-nitroguanidin.

12. Verfahren zur Herstellung der Nitroguanidine der Formel I gemäss Anspruch 1, dadurch gekennzeichnet, dass man entweder

a) ein Amin der Formel II

H-$NR_1$-$CHR_2$-A (II)

mit einem Nitroisothioharnstoff der Formel III

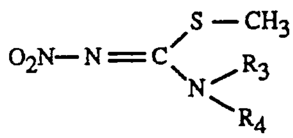

(III),

worin $R_1$, $R_2$, $R_3$, $R_4$ und A die unter Formel I gegebenen Bedeutungen haben, umsetzt, oder

b) eine Verbindung der Formel IV

Hal-$CHR_2$-A (IV)

in Gegenwart einer Base mit einem Nitroguanidinderivat der Formel V

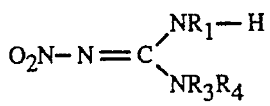

(V),

worin $R_1$, $R_2$, $R_3$, $R_4$ und A die unter Formel I gegebenen Bedeutungen haben und Hal für Halogen, vorzugsweise Chlor oder Brom, steht, umsetzt.

13. Verfahren zur Herstellung der Verbindungen der Formel I, worin $R_3$ und $R_4$ Wasserstoff bedeuten, dadurch gekennzeichnet, dass man ein Amin der Formel II

H-$NR_1$-$CHR_2$-A (II),

worin $R_1$, $R_2$ und A die unter Formel I gegebenen Bedeutungen haben, entweder mit 1-Methyl-1-nitroso-2-nitroguanidin der Formel VI

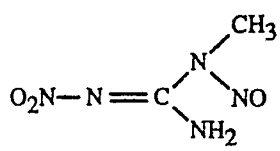

(VI)

oder mit Nitroguanidin der Formel VI

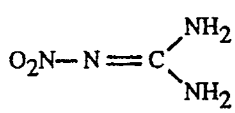

(VII)

umsetzt.

14. Schädlingsbekämpfungsmittel, welches als aktive Komponente mindestens ein Nitroguanidin der Formel I

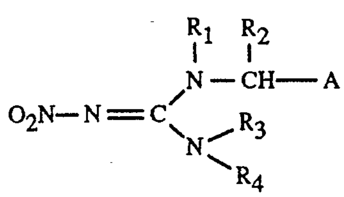

(I)

worin

$R_1$ für Wasserstoff, $C_1$-$C_4$-Alkyl oder $C_3$-$C_6$-Cycloalkyl,

$R_2$ für Wasserstoff oder $C_1$-$C_4$-Alkyl,
$R_3$ für Wasserstoff, $C_1$-$C_4$-Alkyl oder $C_3$-$C_6$-Cycloalkyl,
$R_4$ für Wasserstoff, $C_1$-$C_4$-Alkyl, $C_3$-$C_6$-Cycloalkyl oder ein Rest -$CHR_5$-B, oder
$R_3$ und $R_4$ gemeinsam für -$(CH_2)_4$- oder -$(CH_2)_5$-,
$R_5$ für Wasserstoff oder $C_1$-$C_4$-Alkyl,
A für einen unsubstituierten oder ein- bis vierfach substituierten aromatischen oder nichtaromatischen, monocyclischen oder bicyclischen heterocyclischen Rest, wobei ein bis zwei Substituenten aus der Gruppe $C_1$-$C_3$-Halogenalkyl, Cyclopropyl, Halogencyclopropyl, $C_2$-$C_3$-Alkenyl, $C_2$-$C_3$-Alkinyl, $C_2$-$C_3$-Halogenalkenyl, $C_2$-$C_3$-Halogenalkinyl, $C_1$-$C_3$-Halogenalkoxy, $C_1$-$C_3$-Alkylthio, $C_1$-$C_3$-Halogenalkylthio, Allyloxy, Propargyloxy, Allylthio, Propargylthio, Halogenallyloxy, Halogenallylthio, Cyan und Nitro und ein bis vier Substituenten aus der Gruppe $C_1$-$C_3$-Alkyl, $C_1$-$C_3$-Alkoxy und Halogen ausgewählt sind, und
B für Phenyl, Cyanophenyl, Nitrophenyl, Halogenphenyl mit 1 bis 3 Halogenatomen, 3-Pyridyl, 5-Thiazolyl, durch ein bis zwei Substituenten aus der Gruppe $C_1$-$C_3$-Alkyl, $C_1$-$C_3$-Halogenalkyl, Cyclopropyl, Halogencyclopropyl, $C_2$-$C_3$-Alkenyl, $C_2$-$C_3$-Alkinyl, $C_1$-$C_3$-Alkoxy, $C_2$-$C_3$-Halogenalkenyl, $C_2$-$C_3$-Halogenalkinyl, $C_1$-$C_3$-Halogenalkoxy, $C_1$-$C_3$-Alkylthio, $C_1$-$C_3$-Halogenalkylthio, Allyloxy, Propargyloxy, Allylthio, Propargylthio, Halogenallyloxy, Halogenallylthio, Halogen, Cyan und Nitro substituiertes 5-Thiazolyl; oder durch ein bis zwei Reste aus der Gruppe $C_1$-$C_3$-Halogenalkyl, Cyclopropyl, Halogencyclopropyl, $C_2$-$C_3$-Alkenyl, $C_2$-$C_3$-Alkinyl, $C_2$-$C_3$-Halogenalkenyl, $C_2$-$C_3$-Halogenalkinyl, $C_1$-$C_3$-Halogenalkoxy, $C_1$-$C_3$-Alkylthio, $C_1$-$C_3$-Halogenalkylthio, Allyloxy, Propargyloxy, Allylthio, Propargylthio, Halogenallyloxy, Halogenallylthio, Cyan und Nitro oder durch ein bis vier Reste aus der Gruppe $C_1$-$C_3$-Alkyl, $C_1$-$C_3$-Alkoxy oder Halogen substituiertes 3-Pyridyl stehen; sowie deren Salze mit anorganischen Säuren enthält.

15. Mittel gemäss Anspruch 14, dadurch gekennzeichnet, dass es neben dem Wirkstoff der Formel I noch mindestens einen Trägerstroff enthält.

16. Verfahren zur Bekämpfung von tier- und pflanzenschädigenden Insekten und Arachniden, dadurch gekennzeichnet, dass man die Schädlinge oder ihren Lebensraum mit einer wirksamen Menge eines Nitroguanidins der Formel I

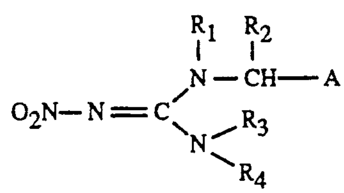

(I)

worin
$R_1$ für Wasserstoff, $C_1$-$C_4$-Alkyl oder $C_3$-$C_6$-Cycloalkyl,
$R_2$ für Wasserstoff oder $C_1$-$C_4$-Alkyl,
$R_3$ für Wasserstoff, $C_1$-$C_4$-Alkyl oder $C_3$-$C_6$-Cycloalkyl,
$R_4$ für Wasserstoff, $C_1$-$C_4$-Alkyl, $C_3$-$C_6$-Cycloalkyl oder ein Rest -$CHR_5$-B, oder
$R_3$ und $R_4$ gemeinsam für -$(CH_5)_4$- oder -$(CH_2)_5$-,
$R_5$ für Wasserstoff oder $C_1$-$C_4$-Alkyl,
A für einen unsubstituierten oder ein- bis vierfach substituierten aromatischen oder nichtaromatischen, monocyclischen oder bicyclischen heterocyclischen Rest, wobei ein bis zwei Substituenten aus der Gruppe $C_1$-$C_3$-Halogenalkyl, Cyclopropyl, Halogencyclopropyl, $C_2$-$C_3$-Alkenyl, $C_2$-$C_3$-Alkinyl, $C_2$-$C_3$-Halogenalkenyl; $C_2$-$C_3$-Halogenalkinyl, $C_1$-$C_3$-Halogenalkoxy, $C_1$-$C_3$-Alkylthio, $C_1$-$C_3$-Halogenalkylthio, Allyloxy, Propargyloxy, Allylthio, Propargylthio, Halogenallyloxy, Halogenallylthio, Cyan und Nitro und ein bis vier Substituenten aus der Gruppe $C_1$-$C_3$-Alkyl, $C_1$-$C_3$-Alkoxy und Halogen ausgewählt sind, und
B für Phenyl, Cyanophenyl, Nitrophenyl, Halogenphenyl mit 1 bis 3 Halogenatomen, 3-Pyridyl, 5-Thiazolyl, durch ein bis zwei Substituenten aus der Gruppe $C_1$-$C_3$-Alkyl, $C_1$-$C_3$-Halogenalkyl, Cyclopropyl, Halogencyclopropyl, $C_2$-$C_3$-Alkenyl, $C_2$-$C_3$-Alkinyl, $C_1$-$C_3$-Alkoxy, $C_2$-$C_3$-Halogenalkenyl, $C_2$-$C_3$-Halogenalkinyl, $C_1$-$C_3$-Halogenalkoxy, $C_1$-$C_3$-Alkylthio, $C_1$-$C_3$-Halogenalkylthio, Allyloxy, Propargyloxy, Allylthio, Propargylthio, Halogenallyloxy, Halogenallylthio, Halogen, Cyan und Nitro substituiertes 5-Thiazolyl; oder durch ein bis zwei Reste aus der Gruppe $C_1$-$C_3$-Halogenalkyl, Cyclopropyl, Halogencyclopropyl, $C_2$-$C_3$-Alkenyl, $C_2$-$C_3$-Alkinyl, $C_2$-$C_3$-Halogenalkenyl, $C_2$-$C_3$-Halogenalkinyl, $C_1$-$C_3$-Halogenalkoxy, $C_1$-$C_3$-Alkylthio, $C_1$-$C_3$-Halogenalkylthio, Allyloxy, Propargyloxy, Allylthio, Propargylthio, Halogenallyloxy, Halogenallylthio, Cyan und Nitro oder durch ein bis vier Reste aus der Gruppe $C_1$-$C_3$-Alkyl, $C_1$-$C_3$-Alkoxy oder Halogen substituiertes 3-Pyridyl stehen; sowie deren Salze mit anorganischen Säuren behandelt.

17. Verwendung eines Nitroguanidins der Formel I

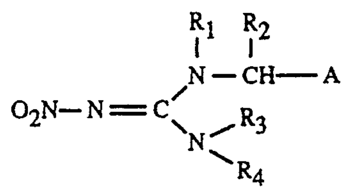

(I)

worin

$R_1$ für Wasserstoff, $C_1$-$C_4$-Alkyl oder $C_3$-$C_6$-Cycloalkyl,

$R_2$ für Wasserstoff oder $C_1$-$C_4$-Alkyl,

$R_3$ für Wasserstoff, $C_1$-$C_4$-Alkyl oder $C_3$-$C_6$-Cycloalkyl,

$R_4$ für Wasserstoff, $C_1$-$C_4$-Alkyl, $C_3$-$C_6$-Cycloalkyl oder ein Rest $-CHR_5$-B, oder

$R_3$ und $R_4$ gemeinsam für $-(CH_2)_4-$ oder $-(CH_2)_5-$,

$R_5$ für Wasserstoff oder $C_1$-$C_4$-Alkyl,

A für einen unsubstituierten oder ein- bis vierfach substituierten aromatischen oder nichtaromatischen, monocyclischen oder bicyclischen heterocyclischen Rest, wobei ein bis zwei Substituenten aus der Gruppe $C_1$-$C_3$-Halogenalkyl, Cyclopropyl, Halogencyclopropyl, $C_2$-$C_3$-Alkenyl, $C_2$-$C_3$-Alkinyl, $C_2$-$C_3$-Halogenalkenyl, $C_2$-$C_3$-Halogenalkinyl, $C_1$-$C_3$-Halogenalkoxy, $C_1$-$C_3$-Alkylthio, $C_1$-$C_3$-Halogenalkylthio, Allyloxy, Propargyloxy, Allylthio, Propargylthio, Halogenallyloxy, Halogenallylthio, Cyan und Nitro und ein bis vier Substituenten aus der Gruppe $C_1$-$C_3$-Alkyl, $C_1$-$C_3$-Alkoxy und Halogen ausgewählt sind, und

B für Phenyl, Cyanophenyl, Nitrophenyl, Halogenphenyl mit 1 bis 3 Halogenatomen, 3-Pyridyl, 5-Thiazolyl, durch ein bis zwei Substituenten aus der Gruppe $C_1$-$C_3$-Alkyl, $C_1$-$C_3$-Halogenalkyl, Cyclopropyl, Halogencyclopropyl, $C_2$-$C_3$-Alkenyl, $C_2$-$C_3$- Alkinyl, $C_1$-$C_3$-Alkoxy, $C_2$-$C_3$-Halogenalkenyl, $C_2$-$C_3$-Halogenalkinyl, $C_1$-$C_3$-Halogenalkoxy, $C'$-$C_3$-Alkylthio, $C_1$-$C_3$-Halogenalkylthio, Allyloxy, Propargyloxy, Allylthio, Propargylthio, Halogenallyloxy, Halogenallylthio, Halogen, Cyan und Nitro substituiertes 5-Thiazolyl; oder durch ein bis zwei Reste aus der Gruppe $C_1$-$C_3$-Halogenalkyl, Cyclopropyl, Halogencyclopropyl, $C_2$-$C_3$-Alkenyl, $C_2$-$C_3$-Alkinyl, $C_2$-$C_3$-Halogenalkenyl, $C_2$-$C_3$-Halogenalkinyl, $C_1$-$C_3$-Halogenalkoxy, $C_1$-$C_3$-Alkylthio, $C_1$-$C_3$-Halogenalkylthio, Allyloxy, Propargyloxy, Allylthio, Propargylthio, Halogenallyloxy, Halogenallylthio, Cyan und Nitro oder durch ein bis vier Reste aus der Gruppe $C_1$-$C_3$-Alkyl, $C_1$-$C_3$-Alkoxy oder Halogen substituiertes 3-Pyridyl stehen; sowie dessen Salzen mit anorganischen Säuren zur Bekämpfung von Schädlingen an Tieren und Pflanzen.

18. Verwendung gemäss Anspruch 17, dadurch gekennzeichnet, dass es sich bei den Schädlingen um pflanzenschädigende Insekten und Arachniden handelt.

Patentansprüche für folgenden Vertragsstaat ES:

1. Schädlingsbekämpfungsmittel, welches als aktive Komponente mindestens ein Nitroguanidin der Formel I

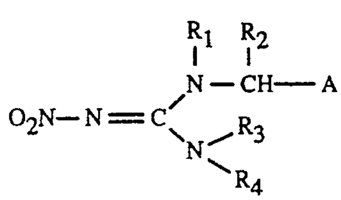

(I)

worin

$R_1$ für Wasserstoff, $C_1$-$C_4$-Alkyl oder $C_3$-$C_6$-Cycloalkyl,

$R_2$ für Wasserstoff oder $C_1$-$C_4$-Alkyl,

$R_3$ für Wasserstoff, $C_1$-$C_4$-Alkyl oder $C_3$-$C_6$-Cycloalkyl,

$R_4$ für Wasserstoff, $C_1$-$C_4$-Alkyl, $C_3$-$C_6$-Cycloalkyl oder ein Rest $-CHR_5$-B, oder

$R_3$ und $R_4$ gemeinsam für $-(CH_5)_4-$ oder $-(CH_2)_5-$,

$R_5$ für Wasserstoff oder $C_1$-$C_4$-Alkyl,

A für einen unsubstituierten oder ein- bis vierfach substituierten aromatischen oder nichtaromatischen, monocyclischen oder bicyclischen heterocyclischen Rest, wobei ein bis zwei Substituenten aus der Gruppe $C_1$-$C_3$-Halogenalkyl, Cyclopropyl, Halogencyclopropyl, $C_2$-$C_3$-Alkenyl, $C_2$-$C_3$-Alkinyl, $C_2$-$C_3$-Halogenalkenyl, $C_2$-$C_3$-Halogenalkinyl, $C_1$-$C_3$-Halogenalkoxy, $C_1$-$C_3$-Alkylthio, $C_1$-$C_3$-Halogenalkylthio, Allyloxy, Propargyloxy, Allylthio, Propargylthio, Halogenallyloxy, Halogenallylthio, Cyan und Nitro und ein bis vier Substituenten aus der Gruppe $C_1$-$C_3$-Alkyl, $C_1$-$C_3$-Alkoxy und Halogen ausgewählt sind, und

B für Phenyl, Cyanophenyl, Nitrophenyl, Halogenphenyl mit 1 bis 3 Halogenatomen, 3-Pyridyl, 5-Thiazolyl, durch ein bis zwei Substituenten aus der Gruppe $C_1$-$C_3$-Alkyl, $C_1$-$C_3$-Halogenalkyl, Cyclopropyl, Halogencyclopropyl, $C_2$-$C_3$-Alkenyl, $C_2$-$C_3$-Alkinyl, $C_1$-$C_3$-Alkoxy, $C_2$-$C_3$-Halogenalkenyl, $C_2$-$C_3$-Halogenalkinyl, $C_1$-$C_3$-Halogenalkoxy, $C_1$-$C_3$-Alkylthio, $C_1$-$C_3$-Halogenalkylthio, Allyloxy, Propargyloxy, Allylthio, Propargylthio, Halogenallyloxy, Halogenallylthio, Halogen, Cyan und Nitro substituiertes 5-Thiazolyl; oder durch ein bis zwei Reste aus der Gruppe $C_1$-$C_3$-Halogenalkyl, Cyclopropyl, Halogencyclopropyl, $C_2$-$C_3$-Alkenyl, $C_2$-$C_3$-Alkinyl, $C_2$-$C_3$-Halogenalkenyl, $C_2$-$C_3$-Halogenalkinyl, $C_1$-$C_3$-Halogenalkoxy, $C_1$-$C_3$-Alkylthio, $C_1$-$C_3$-Halogenalkylthio, Allyloxy, Propargyloxy, Allylthio, Propargylthio, Halogenallyloxy, Halogenallylthio, Cyan und Nitro oder durch ein bis vier Reste aus der Gruppe $C_1$-$C_3$-Alkyl, $C_1$-$C_3$-Alkoxy oder Halogen substituiertes 3-Pyridyl stehen; sowie deren Salze mit anorganischen Säuren enthält.

2. Mittel gemäss Anspruch 1, dadurch gekennzeichnet, dass der heterocyclische Rest A ungesättigt ist, über ein Kohlenstoffatom an den Grundkörper des Nitroguanidins der Formel I gebunden ist und mindestens ein Stickstoffatom enthält.

3. Mittel gemäss Anspruch 2, dadurch gekennzeichnet, dass der heterocyclische Rest A ungesättigt ist und über ein Kohlenstoffatom an den Grundkörper des Nitroguanidins gebunden ist und ein bis drei Heteroatome aus der Reihe Sauerstoff, Schwefel und Stickstoff enthält, wobei jeweils höchstens ein Sauerstoff- oder Schwefelatom enthalten ist.

4. Mittel gemäss Anspruch 3, dadurch gekennzeichnet, dass der heterocyclische Rest A ein bis drei Heteroatome aus der Reihe Sauerstoff, Schwefel und Stickstoff enthält, wovon stets ein Heteroatom Stickstoff ist und höchstens ein Sauerstoffatom oder Schwefelatom enthalten ist.

5. Mittel gemäss Anspruch 1, dadurch gekennzeichnet, dass der heterocyclische Rest A einen über ein Kohlenstoffatom an den Guanidinkörper gebundenen Grundkörper aus der Gruppe

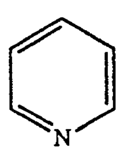 , 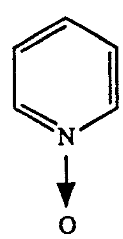 , 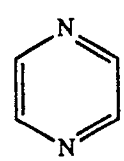 , 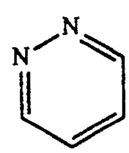 , 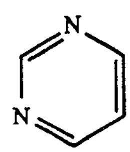 , 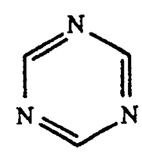 , 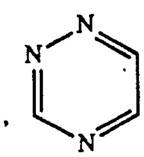 ,

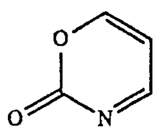, 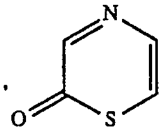, 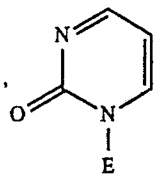, 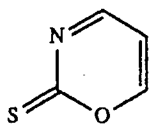, 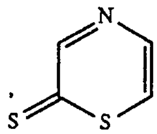, 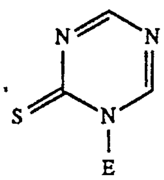,

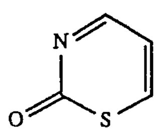, 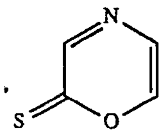, 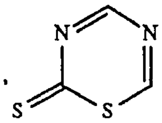, 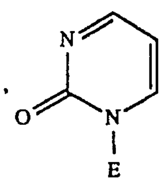, 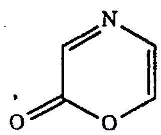,

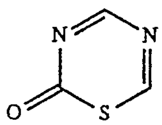, 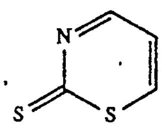, 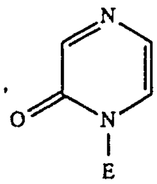, 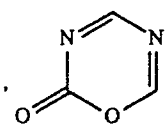, 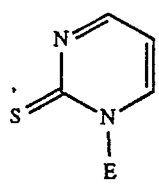, 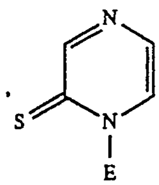,

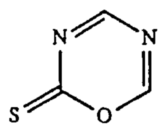, 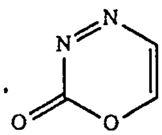, 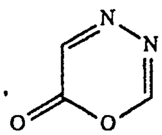, 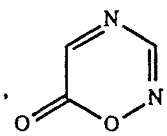, 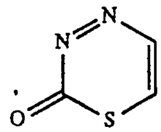,

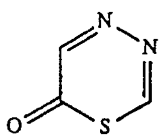, 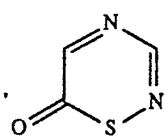, 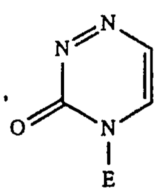, 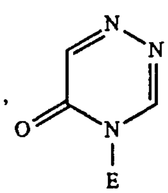, 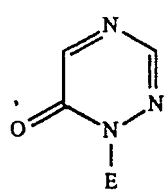,

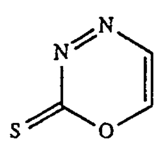, 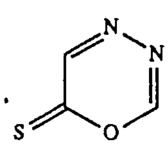, 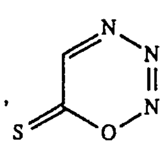, 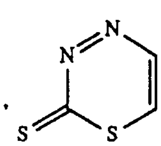, 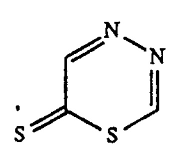,

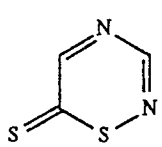, 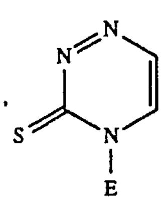, 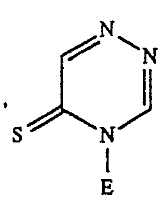, 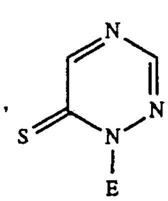, 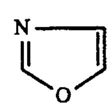,

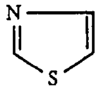 , 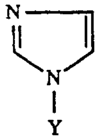 , 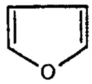 , 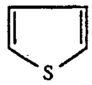 ,

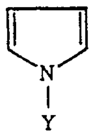 , 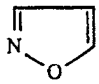 , 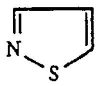 , 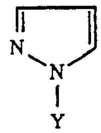 ,

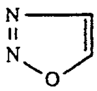 , 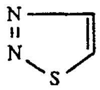 , 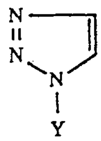 , 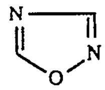 , 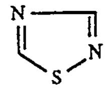 ,

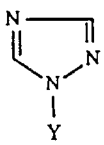 , 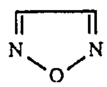 , 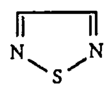 , 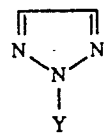 , 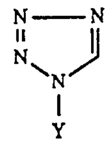 ,

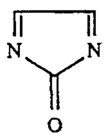 , 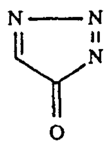 , 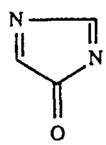 , 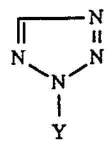 , 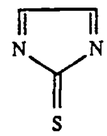 ,

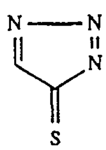 , 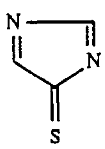 , 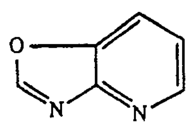 , 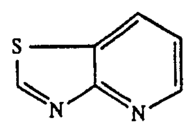 , 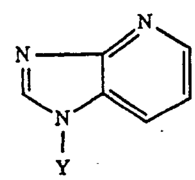 ,

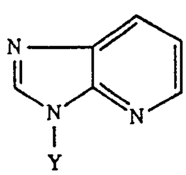 , 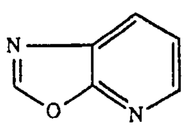 , 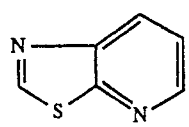 , 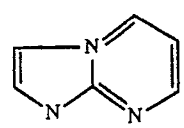 ,

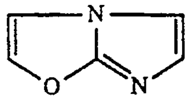, 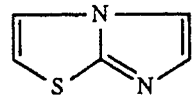, 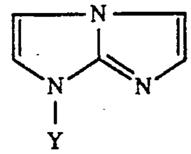,

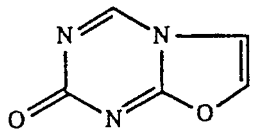, 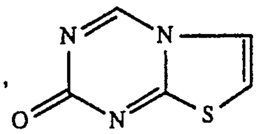, 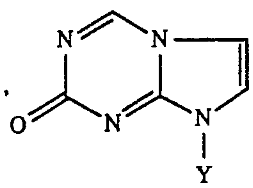, 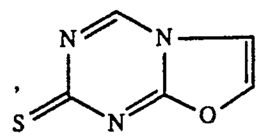,

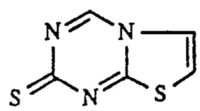 und 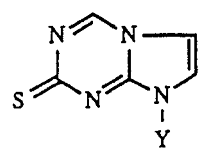

darstellt, welcher unsubstituiert ist oder je nach den Substitutionsmöglichkeiten des Ringsystems bis zu vier der im Anspruch 1 definierten Substituenten trägt und worin E für $C_1$-$C_3$-Alkyl und Y für Wasserstoff, $C_1$-$C_3$-Alkyl oder Cyclopropyl stehen.

6. Mittel gemäss Anspruch 5, dadurch gekennzeichnet, dass der heterocyclische Rest A unsubstituiert ist oder ein bis drei Substituenten aus der Gruppe Halogen, $C_1$-$C_3$-Alkyl, $C_1$-$C_3$-Halogenalkyl, $C_1$-$C_3$-Halogenalkoxy oder $C_1$-$C_3$-Alkoxy trägt.

7. Mittel gemäss Anspruch 6, dadurch gekennzeichnet, dass die heterocyclischen Reste A Pyridylreste oder Thiazolylreste sind.

8. Mittel gemäss Anspruch 1, dadurch gekennzeichnet, dass der Rest B für einen Phenyl-, Pyridyl- oder Thiazolylrest steht, welcher unsubstituiert oder durch ein bis zwei Reste aus der Gruppe Halogen, $C_1$-$C_3$-Alkyl, $C_1$-$C_3$-Halogenalkyl, $C_1$-$C_3$-Halogenalkoxy oder $C_1$-$C_3$-Alkoxy substituiert ist.

9. Mittel gemäss Anspruch 7, dadurch gekennzeichnet, dass A für 3-Pyridyl, 2-Halogenpyrid-5-yl, 2,3-Dihalogenpyrid-5-yl oder 2-Halogenthiazol-4-yl, 1-Oxopyrid-3-yl, 1-Oxo-2-halogenpyrid-5-yl oder 1-Oxo-2,3-dihalogenpyrid-5-yl steht.

10. Mittel gemäss Anspruch 1, dadurch gekennzeichnet, dass $R_1$ und $R_3$ unabhängig voneinander Wasserstoff, Methyl, Äthyl oder Cyclopropyl, $R_2$ Wasserstoff, $R_4$ Wasserstoff oder Methyl und A Pyridyl, 1-Oxopyridyl, Thiazolyl oder jeweils durch ein bis drei Substituenten aus der Gruppe Halogen, $C_1$-$C_3$-Alkyl, $C_1$-$C_3$-Halogenalkyl, $C_1$-$C_3$-Halogenalkoxy oder $C_1$-$C_3$-Alkoxy substituiertes Pyridyl, 1-Oxopyridyl oder Thiazolyl bedeuten.

11. Mittel gemäss Anspruch 1, enthaltend einen Wirkstoff ausgewählt aus der Gruppe 1-(2-Chlorpyrid-5-ylmethyl)-1-methyl-2-nitroguanidin, 1-(2-Chlorpyrid-5-ylmethyl)-2-nitroguanidin, 1-Methyl-2-nitro-3-(pyrid-3-ylmethyl)-guanidin, 1-Methyl-2-nitro-1-(pyrid-3-ylmethyl)-guanidin, 1,2-Dimethyl-3-nitro-1-(pyrid-3-ylmethyl)-guanidin, 1-(2-Chlorpyrid-5-ylmethyl)-1,2-dimethyl-3-nitroguanidin, 1-(2-Chlorthiazol-5-ylmethyl)-1-methyl-2-nitroguanidin, 1-(2-Chlorpyrid-5-ylmethyl)-2,2-dimethyl-3-nitroguanidin, 1-(2-Chlorpyrid-5-ylmethyl)-2-methyl-3-nitroguanidin, 1,1-Dimethyl-2-nitro-3-(pyrid-3-ylmethyl)-guanidin, 1-Aethyl-2-nitro-3-(pyrid-3-methyl)-guanidin, 1-Aethyl-2(2-chlorpyrid-5-ylmethyl)-3-nitroguanidin, 1-Aethyl-1-methyl-2-(2-chlorpyrid-5-ylmethyl)-3-nitroguanidin, 1-(2-Chlorthiazol-5-ylmethyl)-2,2-dimethyl-3-nitroguanidin, 1-(2,3-Dichlorpyrid-5-ylmethyl)-2-methyl-3-nitroguanidin, 1-Aethyl-2-(2,3-dichlorpyrid-5-ylmethyl)-3-nitroguanidin, 1-(2,3-Dichlorpyrid-5-ylmethyl)-1-methyl-2-nitroguanidin, 1-Aethyl-1-(2,3dichlorpyrid-5-ylmethyl)-2-nitroguanidin, 1-(2-Chlor-1-oxopyrid-5-ylmethyl)-1-methyl-2-nitroguanidin, 1-Aethyl-1-(2-chlor-1-oxopyrid-5-ylmethyl)-2-nitroguanidin, 1-(2-Chlor-1-oxopyrid-5-ylmethyl)-2-methyl-3-nitroguanidin, 1-Aethyl-1-(2-chlor-1-oxopyrid-5-ylmethyl)-3-nitroguanidin, 1-(2-Chlorthiazol-5-ylmethyl)-2-methyl-3-nitroguanidin, 1-(2-Chlorthiazol-5-ylmethyl)-2-nitroguanidin und 1-Aethyl-2-(2-chlorthiazol-5-ylmethyl)-3-nitroguanidin.

12. Verfahren zur Herstellung der Nitroguanidine der Formel I gemäss Anspruch 1, dadurch gekennzeichnet, dass man entweder

a) ein Amin der Formel II

$H$-$NR_1$-$CHR_2$-$A$ (II)

mit einem Nitroisothiohamstoff der Formel III

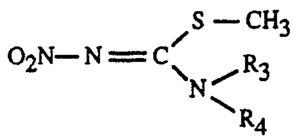

(III),

worin $R_1$, $R_2$, $R_3$, $R_4$ und A die unter Formel I gegebenen Bedeutungen haben, umsetzt, oder
b) eine Verbindung der Formel IV
Hal-$CHR_2$-A (IV)
in Gegenwart einer Base mit einem Nitroguanidinderivat der Formel V

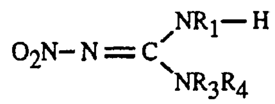

(V),

worin $R_1$, $R_2$, $R_3$, $R_4$ und A die unter Formel I gegebenen Bedeutungen haben und Hal für Halogen, vorzugsweise Chlor oder Brom, steht, umsetzt.
13. Verfahren zur Herstellung der Verbindungen der Formel I, worin $R_3$ und $R_4$ Wasserstoff bedeuten, dadurch gekennzeichnet, dass man ein Amin der Formel II
H-$NR_1$-$CHR_2$-A (II),
worin $R_1$, $R_2$ und A die unter Formel I gegebenen Bedeutungen haben, entweder mit 1-Methyl-1-nitroso-2-nitroguanidin der Formel VI

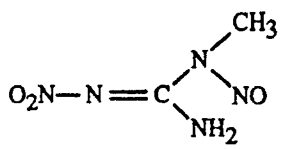

(VI)

oder mit Nitroguanidin der Formel VII

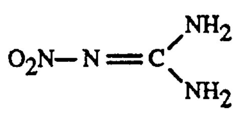

(VII)

umsetzt.
14. Mittel gemäss Anspruch 1, dadurch gekennzeichnet, dass es neben dem Wirkstoff der Formel I noch mindestens einen Trägerstroff enthält.
15. Verfahren zur Bekämpfung von tier- und pflanzenschädigenden Insekten und Arachniden, dadurch gekennzeichnet, dass man die Schädlinge oder ihren Lebensraum mit einer wirksamen Menge eines Nitroguanidins der Formel I

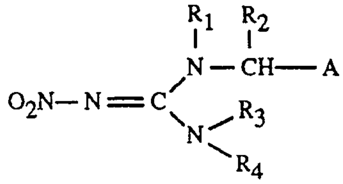

(I)

worin
$R_1$ für Wasserstoff, $C_1$-$C_4$-Alkyl oder $C_3$-$C_6$-Cycloalkyl,

$R_2$ für Wasserstoff oder $C_1$-$C_4$-Alkyl,
$R_3$ für Wasserstoff, $C_1$-$C_4$-Alkyl oder $C_3$-$C_6$-Cycloalkyl,
$R_4$ für Wasserstoff, $C_1$-$C_4$-Alkyl, $C_3$-$C_6$-Cycloalkyl oder ein Rest -$CHR_5$-B, oder
$R_3$ und $R_4$ gemeinsam für -$(CH_2)_4$- oder -$(CH_2)_5$-,
$R_5$ für Wasserstoff oder $C_1$-$C_4$-Alkyl,
A für einen unsubstituierten oder ein- bis vierfach substituierten aromatischen oder nichtaromatischen, monocyclischen oder bicyclischen heterocyclischen Rest, wobei ein bis zwei Substituenten aus der Gruppe $C_1$-$C_3$-Halogenalkyl, Cyclopropyl, Halogencyclopropyl, $C_2$-$C_3$-Alkenyl, $C_2$-$C_3$-Alkinyl, $C_2$-$C_3$-Halogenalkenyl, $C_2$-$C_3$-Halogenalkinyl, $C_1$-$C_3$-Halogenalkoxy, $C_1$-$C_3$-Alkylthio, $C_1$-$C_3$-Halogenalkylthio, Allyloxy, Propargyloxy, Allylthio, Propargylthio, Halogenallyloxy, Halogenallylthio, Cyan und Nitro und ein bis vier Substituenten aus der Gruppe $C_1$-$C_3$-Alkyl, $C_1$-$C_3$-Alkoxy und Halogen ausgewählt sind, und
B für Phenyl, Cyanophenyl, Nitrophenyl, Halogenphenyl mit 1 bis 3 Halogenatomen, 3-Pyridyl, 5-Thiazolyl, durch ein bis zwei Substituenten aus der Gruppe $C_1$-$C_3$-Alkyl, $C_1$-$C_3$-Halogenalkyl, Cyclopropyl, Halogencyclopropyl, $C_2$-$C_3$-Alkenyl, $C_2$-$C_3$-Alkinyl, $C_1$-$C_3$-Alkoxy, $C_2$-$C_3$-Halogenalkenyl, $C_2$-$C_3$-Halogenalkinyl, $C_1$-$C_3$-Halogenalkoxy, $C_1$-$C_3$-Alkylthio, $C_1$-$C_3$-Halogenalkylthio, Allyloxy, Propargyloxy, Allylthio, Propargylthio, Halogenallyloxy, Halogenallylthio, Halogen, Cyan und Nitro substituiertes 5-Thiazolyl; oder durch ein bis zwei Reste aus der Gruppe $C_1$-$C_3$-Halogenaikyl, Cyclopropyl, Halogencyclopropyl, $C_2$-$C_3$-Alkenyl, $C_2$-$C_3$-Alkinyl, $C_2$-$C_3$-Halogenalkenyl, $C_2$-$C_3$-Halogenalkinyl, $C_1$-$C_3$-Halogenalkoxy, $C_1$-$C_3$-Alkylthio, $C_1$-$C_3$-Halogenalkylthio, Allyloxy, Propargyloxy, Allylthio, Propargylthio, Halogenallyloxy, Halogenallylthio, Cyan und Nitro oder durch ein bis vier Reste aus der Gruppe $C_1$-$C_3$-Alkyl, $C_1$-$C_3$-Alkoxy oder Halogen substituiertes 3-Pyridyl stehen; sowie deren Salze mit anorganischen Säuren behandelt.

16. Verwendung eines Nitroguanidins der Formel I

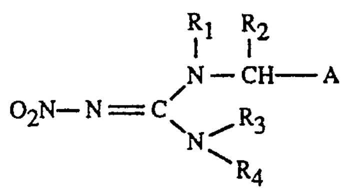

(I)

worin
$R_1$ für Wasserstoff, $C_1$-$C_4$-Alkyl oder $C_3$-$C_6$-Cycloalkyl,
$R_2$ für Wasserstoff oder $C_1$-$C_4$-Alkyl,
$R_3$ für Wasserstoff, $C_1$-$C_4$-Alkyl oder $C_3$-$C_6$-Cycloalkyl,
$R_4$ für Wasserstoff, $C_1$-$C_4$-Alkyl, $C_3$-$C_6$-Cycloalkyl oder ein Rest -$CHR_5$-B, oder
$R_3$ und $R_4$ gemeinsam für -$(CH_2)_4$- oder -$(CH_2)_5$-,
$R_5$ für Wasserstoff oder $C_1$-$C_4$-Alkyl,
A für einen unsubstituierten oder ein- bis vierfach substituierten aromatischen oder nichtaromatischen, monocyclischen oder bicyclischen heterocyclischen Rest, wobei ein bis zwei Substituenten aus der Gruppe $C_1$-$C_3$-Halogenalkyl, Cyclopropyl, Halogencyclopropyl, $C_2$-$C_3$-Alkenyl, $C_2$-$C_3$-Alkinyl, $C_2$-$C_3$-Halogenalkenyl, $C_2$-$C_3$-Halogenalkinyl, $C_1$-$C_3$-Halogenalkoxy, $C_1$-$C_3$-Alkylthio, $C_1$-$C_3$-Halogenalkylthio, Allyloxy, Propargyloxy, Allylthio, Propargylthio, Halogenallyloxy, Halogenallylthio, Cyan und Nitro und ein bis vier Substituenten aus der Gruppe $C_1$-$C_3$-Alkyl, $C_1$-$C_3$-Alkoxy und Halogen ausgewählt sind, und
B für Phenyl, Cyanophenyl, Nitrophenyl, Halogenphenyl mit 1 bis 3 Halogenatomen, 3-Pyridyl, 5-Thiazolyl, durch ein bis zwei Substituenten aus der Gruppe $C_1$-$C_3$-Alkyl, $C_1$-$C_3$-Halogenalkyl, Cyclopropyl, Halogencyclopropyl, $C_2$-$C_3$-Alkenyl, $C_2$-$C_3$-Alkinyl, $C_1$-$C_3$-Alkoxy, $C_2$-$C_3$-Halogenalkenyl, $C_2$-$C_3$-Halogenalkinyl, $C_1$-$C_3$-Halogenalkoxy, $C_1$-$C_3$-Alkylthio, $C_1$-$C_3$-Halogenalkylthio, Allyloxy, Propargyloxy, Allylthio, Propargylthio, Halogenallyloxy, Halogenallylthio, Halogen, Cyan und Nitro substituiertes 5-Thiazolyl; oder durch ein bis zwei Reste aus der Gruppe $C_1$-$C_3$-Halogenalkyl, Cyclopropyl, Halogencyclopropyl, $C_2$-$C_3$-Alkenyl, $C_2$-$C_3$-Alkinyl, $C_2$-$C_3$-Halogenalkenyl, $C_2$-$C_3$-Halogenalkinyl, $C_1$-$C_3$-Halogenalkoxy, $C_1$-$C_3$-Alkylthio, $C_1$-$C_3$-Halogenalkylthio, Allyloxy, Propargyloxy, Allylthio, Propargylthio, Halogenallyloxy, Halogenallylthio, Cyan und Nitro oder durch ein bis vier Reste aus der Gruppe $C_1$-$C_3$-Alkyl, $C_1$-$C_3$-Alkoxy oder Halogen substituiertes 3-Pyridyl stehen; sowie dessen Salzen mit anorganischen Säuren zur Bekämpfung von Schädlingen an Tieren und Pflanzen.

17. Verwendung gemäss Anspruch 16, dadurch gekennzeichnet, dass es sich bei den Schädlingen um pflanzenschädigende Insekten und Arachniden handelt.